(19) Europäisches Patentamt
European Patent Office
Office européen des brevets

(11) EP 1 235 833 B1

(12) **EUROPEAN PATENT SPECIFICATION**

(45) Date of publication and mention of the grant of the patent:
**25.02.2004 Bulletin 2004/09**

(21) Application number: **00984009.1**

(22) Date of filing: **07.12.2000**

(51) Int Cl.[7]: **C07F 9/6561**, A61K 31/675, A61P 31/12

(86) International application number:
**PCT/US2000/033204**

(87) International publication number:
**WO 2001/042256 (14.06.2001 Gazette 2001/24)**

(54) **ANALOGS OF (1S, CIS)-4-(2-AMINO-9H-PURIN-9-YL)-2-CYCLOPENTENE-1-METHANOL AS ANTIVIRAL**

(1S, CIS)-4-(2-AMINO-9H-PURIN-9-YL)-2-CYCLOPENTEN-1-METHANOL ANALOGUE ALS ANTIVIRAL

ANALOGUES DE (1S, cis)-4-(2-AMINO-9H-PURIN-9-YL)-2-CYCLOPENTENE-1-METHANOL EN TANT QU'ANTIVIRAL

(84) Designated Contracting States:
**AT BE CH CY DE DK ES FI FR GB GR IE IT LI LU MC NL PT SE TR**

(30) Priority: **10.12.1999 US 170225 P**

(43) Date of publication of application:
**04.09.2002 Bulletin 2002/36**

(73) Proprietor: **GLAXO GROUP LIMITED**
**Greenford, Middlesex UB6 ONN (GB)**

(72) Inventor: **Daluge, Susan Mary,**
**c/o Glaxo Wellcome Inc.**
**Research Triangle Park, NC 27709 (US)**

(74) Representative: **Crawley, Karen Anne et al**
**GlaxoSmithKline**
**Corporate Intellectual Property (CN9.25.1)**
**980 Great West Road**
**Brentford, Middlesex TW8 9GS (GB)**

(56) References cited:
**EP-A- 0 434 450**
**WO-A-00/18775**
**WO-A-00/47591**
**WO-A-96/29336**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 235 833 B1

## Description

### FIELD OF THE INVENTION

The present invention relates to phosphoramidates of 6-modified analogs of (1S, cis)-4-(2-amino-9H-purin-9-yl)-2-cyclopentene-1-methanol and their use in medical therapy.

### BACKGROUND OF THE INVENTION

Retroviruses form a sub-group of RNA viruses which, in order to replicate, must first "reverse transcribe" the RNA of their genome into DNA ("transcription" conventionally describes the synthesis of RNA from DNA). Once in the form of DNA, the viral genome may be incorporated into the host cell genome, allowing it to take advantage of the host cell's transcription/translation machinery for the purposes of replication. Once incorporated, the viral DNA is virtually indistinguishable from the host's DNA and, in this state, the virus may persist for the life of the cell.

A species of retrovirus, the Human immunodeficiency virus (HIV) has been reproducibly isolated from patients with AIDS (acquired immunodeficiency syndrome) or with the symptoms that frequently precede AIDS. AIDS is an immunosuppressive or immunodestructive disease that predisposes subjects to fatal opportunistic infections. Characteristically, AIDS is associated with a progressive depletion of T-cells, especially the helper-inducer subset bearing the CD4 surface marker. HIV is cytopathic and appears to preferentially infect and destroy T-cells bearing the CD4 marker, and it is now generally recognized that HIV is the etiological agent of AIDS. Clinical conditions such as AIDS-related complex (ARC), progressive generalized lymphadenopathy (PGL), Karposi's sarcoma, thrombocytopenic purpura, AIDS-related neurological conditions, such as AIDS dementia complex, multiple sclerosis or tropical paraparesis, and also anti-HIV antibody-positive and HIV-positive conditions, including such conditions in asymptomatic patients, are also conditions which may be treated by appropriate anti-viral therapy.

Another RNA virus which has been recognized as the causative agent of an increasingly serious international health problem is the non-A, non-B hepatitis virus. At least 80% of cases of chronic post-transfusional non-A, non-B hepatitis have been shown to be due to the virus now identified as hepatitis C and this virus probably accounts for virtually all cases of post-transfusional hepatitis in clinical settings where blood products are screened for hepatitis B. Whereas approximately half of the cases of acute hepatitis C infection resolve spontaneously over a period of months, the remainder become chronic and in many if not all such cases chronic active hepatitis ensues with the potential for cirrhosis and hepatocellular carcinoma. The structure of the hepatitis C virus genome has been elucidated and the virus has been characterized as a single stranded RNA virus with similarities to flaviviruses.

Hepatitis B virus (HBV) is a small DNA containing virus which infects humans. It is a member of the class of closely related viruses known as the hepadnaviruses, each member of which selectively infects either mammalian or avian hosts, such as the woodchuck and the duck. Recent insights into the mechanism of replication of the hepadnavirus genome indicate the importance of reverse transcription of an RNA intermediate, suggesting that the reverse transcriptase is a logical chemotherapeutic target. HBV is a viral pathogen of major world-wide importance. The virus is etiologically associated with primary hepatocellular carcinoma and is thought to cause 80% of the world's liver cancer. Clinical effects of infection with HBV range from headache, fever, malaise, nausea, vomiting, anorexia and abdominal pains. Replication of the virus is usually controlled by the immune response, with a course of recovery lasting weeks or months in humans, but infection may be more severe leading to persistent chronic liver disease outlined above.

U.S. Patent No. 4,916,224 discloses 2',3'-dideoxy-2',3'-didehydro-carbocyclic nucleosides and their use in the treatment of HIV. U.S. Patent No. 5,049,671 discloses 6-substituted purine carbocyclic nucleosides and their use in medical therapy particularly in the treatment of HIV and HBV infections. European patent No. EP 0349242A2 discloses 6-substituted purine carbocyclic nucleosides and their use in medical therapy, particularly in the treatment and prophylaxis of HIV and HBV infections. WO 96/29336 discloses masked monophosphate nucleoside analogues for the treament of HIV.

It has now been discovered that certain phosphoramidates of 6-modified analogs of (1S, cis)-4-(2-amino-9H-purin-9-yl)-2-cyclopentene-1-methanol are useful for the treatment of viral infections, particularly hepatitis B and hepatitis C and retroviral infections, especially HIV. Compounds of the present invention have pharmacokinetic properties which render them advantageous as therapeutic agents.

### SUMMARY OF THE INVENTION

The present invention relates to compounds of formula (1)

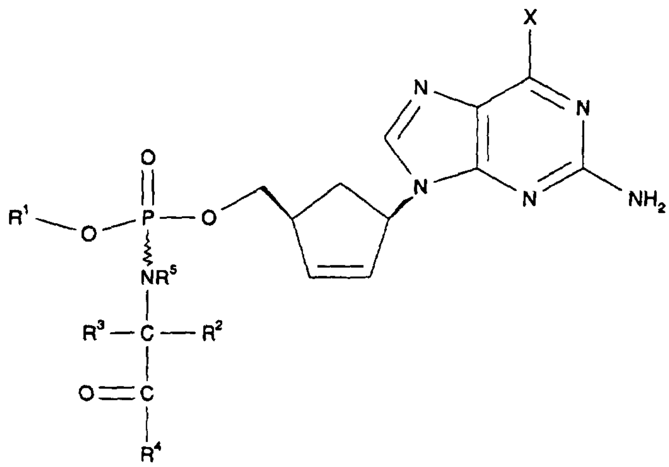

(I)

$R^1$ is hydrogen; $C_{6-14}$aryl; or heteroaryl, optionally substituted with one or more substituents selected from the group consisting of $C_{1-6}$alkoxy, nitro, halogen, amino, hydroxy, carboxylate and esters thereof, carboxyalkyl, -CONHR$^6$, and -CONR$^6$R$^7$, wherein $R^6$ and $R^7$, which may be the same or different, are independently selected from $C_{1-8}$alkyl, $C_1$-$_8$alkylaryl or $C_{6-14}$aryl;

$R^2$ and $R^3$ are independently selected from hydrogen or $C_{1-8}$alkyl, $C_{3-8}$cycloalkyl, $C_{2-8}$alkenyl, $C_{5-8}$cycloalkenyl, $C_{6-14}$aryl, aralkyl wherein each $C_{1-8}$alkyl, $C_{3-8}$cycloalkyl, $C_{2-8}$alkenyl, $C_{5-8}$cycloalkenyl, $C_{6-14}$aryl or aralkyl may be optionally substituted with one or more substituents selected from the group consisting of $C_{1-8}$alkyl, halo, hydroxy, alkoxy, amino, aminoalkyl, aminodialkyl, -SH, thioalkyl, carboxylate and esters thereof, carboxyalkyl, -CONHR$^6$, and -CONR$^6$R$^7$, wherein $R^6$ and $R^7$, which may be the same or different, are independently selected from $C_{1-8}$alkyl, $C_{1-8}$alkylaryl or $C_{6-14}$aryl; or $R^2$ and $R^3$ can together form a 3- to 8-membered ring;

$R^4$ is -OR$^8$, -NR$^8$R$^9$ or -SR$^8$, where $R^8$ and $R^9$, which may be the same or different, are independently selected from hydrogen, or $C_{1-8}$alkyl, $C_{3-8}$cycloalkyl, $C_{2-8}$alkenyl, $C_{5-8}$cycloalkenyl, or aralkyl, wherein each $C_{1-8}$alkyl, $C_{3-8}$cycloalkyl, $C_{2-8}$alkenyl, $C_{5-8}$cycloalkenyl, or aralkyl may be optionally substituted with one or more substituents selected from the group consisting of halo, hydroxy, alkoxy, amino, aminoalkyl, aminodialkyl, -SH, thioalkyl, carboxylate and esters thereof, carboxyalkyl, -CONHR$^6$, and -CONR$^6$R$^7$, wherein $R^6$ and $R^7$, which may be the same or different, are independently selected from $C_{1-8}$alkyl, $C_{1-8}$alkylaryl or $C_{6-14}$aryl;

$R^5$ is hydrogen; $C_{1-8}$alkyl; or $C_{6-14}$aryl; or $R^2$ and $R^5$ may together form a 5- or 6-membered ring; or $R^3$ and $R^5$ may together form a 5- or 6-membered ring;

X is $C_{1-6}$alkoxy, optionally substituted by $C_{3-6}$cycloaklyl; $C_{3-8}$cycloalkyloxy; aryloxy, aralkyl or aralkyloxy in which the aryl may optionally be substituted with $C_{1-8}$alkyl, hydroxy, or halogen; $C_{3-6}$cycloalkylthio; $C_{1-8}$alkylthio; arylthio, or aralkylthio in which the aryl may optionally be substituted with $C_{1-8}$alkyl, hydroxy, or halogen; or X is a heterocyclic group containing an oxygen atom or one or two nitrogen atoms, and 3-7 carbon atoms with optional double bonds in the ring, optionally containing a sulfur and/or oxygen heteroatom and optionally substituted on the ring by one or more $C_{1-8}$alkyl, hydroxy or halogen groups, $C_{3-6}$cycloalkylthio, aralkylthio in which the aryl may be substituted with $C_{1-8}$alkyl, hydroxy or halogen; or X represents an imidazolylthio group in which the imidazolyl moiety may be substituted with $C_{1-8}$alkyl and/or C substituted with nitro; or X represents an amino group which is mono- or di-substituted by $C_{1-8}$alkyl, $C_{1-6}$alkoxy, hydroxy$C_{1-8}$alkyl and/or $C_{3-6}$cycloalkyl, $C_{6-14}$aryl, aralkyl, in which the $C_{6-14}$aryl may be optionally substituted with $C_{1-8}$alkyl, hydroxy , halogen or allyl optionally substituted with mono- or di-alkyl or alkoxy groups;

or a pharmaceutically acceptable derivative thereof; provided that X cannot be amino monosubstituted with cyclopropyl. The present invention relates to the use of compounds of formula (1) in the treatment of viral infections.

The present invention features compounds of formula (1)

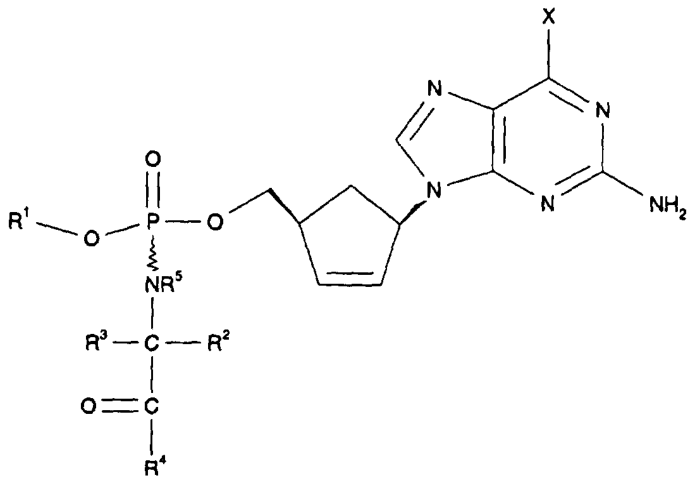

(I)

wherein:

$R^1$ is hydrogen; $C_{6-14}$aryl; or heteroaryl, optionally substituted with one or more substituents selected from the group consisting of $C_{1-6}$alkoxy, nitro, halogen, amino, hydroxy, carboxylate and esters thereof, carboxyalkyl, -CONHR$^6$, and -CONR$^6$R$^7$, wherein $R^6$ and $R^7$, which may be the same or different, are independently selected from $C_{1-8}$alkyl, $C_{1-8}$alkylaryl or $C_{6-14}$aryl;

$R^2$ and $R^3$ are independently selected from hydrogen or $C_{1-8}$alkyl, $C_{3-8}$cycloalkyl, $C_{2-8}$alkenyl, $C_{5-8}$cycloalkenyl, $C_{6-14}$aryl, aralkyl wherein each $C_{1-8}$alkyl, $C_{3-8}$cycloalkyl, $C_{2-8}$alkenyl, $C_{5-8}$cycloalkenyl, $C_{6-14}$aryl or aralkyl may be optionally substituted with one or more substituents selected from the group consisting of $C_{1-8}$alkyl, halo, hydroxy, alkoxy, amino, aminoalkyl, aminodialkyl, -SH, thioalkyl, carboxylate and esters thereof, carboxyalkyl, -CONHR$^6$, and -CONR$^6$R$^7$, wherein $R^6$ and $R^7$, which may be the same or different, are independently selected from $C_{1-8}$alkyl, $C_{1-8}$alkylaryl or $C_{6-14}$aryl; or $R^2$ and $R^3$ can together form a 3- to 8-membered ring;

$R^4$ is -OR$^8$, -NR$^8$R$^9$ or -SR$^8$, where $R^8$ and $R^9$, which may be the same or different, are independently selected from hydrogen, or $C_{1-8}$alkyl, $C_{3-8}$cycloalkyl, $C_{2-8}$alkenyl, $C_{5-8}$cycloalkenyl, or aralkyl, wherein each $C_{1-8}$alkyl, $C_{3-8}$cycloalkyl, $C_{2-8}$alkenyl, $C_{5-8}$cycloalkenyl, or aralkyl may be optionally substituted with one or more substituents selected from the group consisting of halo, hydroxy, alkoxy, amino, aminoalkyl, aminodialkyl, -SH, thioalkyl, carboxylate and esters thereof, carboxyalkyl, -CONHR$^6$, and -CONR$^6$R$^7$, wherein $R^6$ and $R^7$, which may be the same or different, are independently selected from $C_{1-8}$alkyl, $C_{1-8}$alkylaryl or $C_{6-14}$aryl;

$R^5$ is hydrogen; $C_{1-8}$alkyl; or $C_{6-14}$aryl; or $R^2$ and $R^5$ may together form a 5- or 6-membered ring; or $R^3$ and $R^5$ may together form a 5- or 6-membered ring;

X is $C_{1-6}$alkoxy, optionally substituted by $C_{3-6}$cycloaklyl; $C_{3-8}$cycloalkyloxy; aryloxy, aralkyl or aralkyloxy in which the aryl may optionally be substituted with $C_{1-8}$alkyl, hydroxy, or halogen; $C_{3-6}$cycloalkylthio; $C_{1-8}$alkylthio; arylthio, or aralkylthio in which the aryl may optionally be substituted with $C_{1-8}$alkyl, hydroxy, or halogen; or X is a heterocyclic group containing an oxygen atom or one or two nitrogen atoms, and 3-7 carbon atoms with optional double bonds in the ring, optionally containing a sulfur and/or oxygen heteroatom and optionally substituted on the ring by one or more $C_{1-8}$alkyl, hydroxy or halogen groups, $C_{3-6}$cycloalkylthio, aralkylthio in which the aryl may be substituted with $C_{1-8}$alkyl, hydroxy or halogen; or X represents an imidazolylthio group in which the imidazolyl moiety may be substituted with $C_{1-8}$alkyl and/or C substituted with nitro; or X represents an amino group which is mono- or di-substituted by $C_{1-8}$alkyl, $C_{1-6}$alkoxy, hydroxy$C_{1-8}$alkyl and/or $C_{3-6}$cycloalkyl, $C_{6-14}$aryl, aralkyl, in which the $C_{6-14}$aryl may be optionally substituted with $C_{1-8}$alkyl, hydroxy, halogen or allyl optionally substituted with mono- or di-alkyl or alkoxy groups;

or a pharmaceutically acceptable derivative thereof; provided that X cannot be amino monosubstituted with cyclopropyl.

The compounds of the present invention include diastereomers differine in the absolute configuration at phosphorus. Diastereomers may be present as a single isomer or as mixtures of diastereomers.

Where $R^2$ and $R^3$ are different, the L-configuration of naturally occurring amino acids is preferred.

The term "alkyl" refers to a straight-chain or branched-chain saturated aliphatic hydrocarbon radical containing the specified number of carbon atoms, or where no number is specified, preferably from 1 to about 10, more preferably from 1 to about 8 carbon atoms. Examples of alkyl radicals include, but are not limited to, methyl, ethyl, n-propyl, isopropyl, n-butyl, isobutyl, sec-butyl, tert-butyl, pentyl, isoamyl, n-hexyl and the like.

The term "aryl" refers to a carbocyclic aromatic radical (such as phenyl or naphthyl) containing the specified number of carbon atoms, preferably from 6-14 carbon atoms, and more preferably from 6-10 carbon atoms, optionally substituted with one or more substitutents selected from $C_{1-6}$ alkoxy (for example, methoxy), nitro, halogen (for example chloro), amino, carboxylate and hydroxy. Examples of aryl radicals include, but are not limited to phenyl, naphthyl, indenyl, indanyl, azulenyl, fluorenyl, anthracenyl and the like.

The term "alkenyl," alone or in combination with any other term, refers to a straight-chain or branched-chain mono- or poly-unsaturated aliphatic hydrocarbon radical containing the specified number of carbon atoms, or where no number is specified, preferably from 2-10 carbon atoms and more preferably, from 2-6 carbon atoms. Examples of alkenyl radicals include, but are not limited to, ethenyl, propenyl, isopropenyl, butenyl, isobutyenyl, pentenyl, hexenyl, hexadienyl and the like.

The term "alkoxy" refers to an alkyl ether radical, wherein the term "alkyl" is defined above. Examples of suitable alkyl ether radicals include, but are not limited to, methoxy, ethoxy, n-propoxy, isopropoxy, n-butoxy, isobutoxy, sec-butoxy, tert-butoxy and the like, with methoxy being preferred.

The term "halo" or "halogen" refers to a radical of fluorine, chlorine, bromine or iodine.

The term "heterocycle", alone or in combination with another term, refers to a stable 3-7 membered monocyclic heterocyclic ring or 8-11 membered bicyclic heterocyclic ring which is either saturated or unsaturated, and which may be optionally benzofused if monocyclic. Each heterocycle consists of one or more carbon atoms and from one to four heteroatoms selected from the group consisting of nitrogen, oxygen and sulfur. As used herein, the terms "nitrogen and sulfur heteroatoms" include any oxidized form of nitrogen and sulfur, and the quaternized form of any basic nitrogen. A heterocyclyl radical may be attached at any endocyclic carbon or heteroatom which results in the creation of a stable structure. Preferred heterocycles include 5-7 membered monocyclic heterocycles and 8-10 membered bicyclic heterocycles. Examples of such groups include imidazolyl, imidazolinoyl, imidazolidinyl, quinolyl, isoqinolyl, indolyl, indazolyl, indazolinolyl, perhydropyridazyl, pyridazyl, pyridyl, pyrrolyl, pyrrolinyl, pyrrolidinyl, pyrazolyl, pyrazinyl, quinoxolyl, piperidinyl, pyranyl, pyrazolinyl, piperazinyl, pyrimidinyl, pyridazinyl, morpholinyl, thiamorpholinyl, furyl, thienyl, triazolyl, thiazolyl, carbolinyl, tetrazolyl, thiazolidinyl, benzofuranoyl, thiamorpholinyl sulfone, oxazolyl, benzoxazolyl, oxopiperidinyl, oxopyrrolidinyl, oxoazepinyl, azepinyl, isoxozolyl, isothiazolyl, furazanyl, tetrahydropyranyl, tetrahydrofuranyl, thiazolyl, thiadiazoyl, dioxolyl, dioxinyl, oxathiolyl, benzodioxolyl, dithiolyl, thiophenyl, tetrahydrothiophenyl, sulfolanyl, dioxanyl, dioxolanyl, tetahydrofurodihydrofuranyl, tetrahydropyranodihydrofuranyl, dihydropyranyl, tetradyrofurofuranyl and tetrahydropyranofuranyl.

The term "pharmaceutically acceptable derivative", as used herein, means any pharmaceutically acceptable salt, ester, salt of an ester, or other derivative of a compound of this invention which, upon administration to a recipient, is capable of providing (directly or indirectly) a compound of this invention or an inhibitorily active metabolite or residue thereof. Particularly favored derivatives and prodrugs are those that increase the bioavailability of the compounds of this invention when such compounds are administered to a mammal (e.g., by allowing an orally administered compound to be more readily absorbed into the blood) or which enhance delivery of the parent compound to a biological compartment (e.g., the brain or lymphatic system) relative to the parent species.

Compounds of formula (I) and their pharmaceutically acceptable derivatives may hereinafter be referred to as compounds according to the invention.

A further aspect of the present invention features a compound of formula (1) wherein R' is $C_{6-14}$aryl; $R^2$ and $R^3$ are independently selected from hydrogen, $C_{1-8}$alkyl, $C_{6-14}$aryl or aralkyl optionally substituted with $C_{1-8}$alkyl; $R^4$ is $-OR^8$, where $R^8$ is selected from $C_{1-8}$alkyl or aralkyl; $R^5$ is hydrogen; and X represents $C_{1-6}$alkoxy or $C_{1-6}$alkylthio; or X represents a heterocyclic group containing a nitrogen atom, and 3-7 carbon atoms; or X represents an amino group which is mono- or di-substituted by $C_{1-6}$alkyl, $C_{1-6}$alkoxy, hydroxy$C_{1-6}$alkyl and/or $C_{3-6}$cycloakyl), $C_{6-14}$aryl, aralkyl, in which the aryl may be optionally substituted with $C_{1-8}$alkyl, hydroxy, halogen or allyl optionally substituted with mono- or di-alkyl or alkoxy groups; or a pharmaceuticallly acceptable derivative thereof; provided that X cannot be amino monosubstituted with cyclopropyl.

Another aspect of the present invention features compounds of formula (I) selected from the group consisting of

(1S,*cis*)-4-[2-Amino-6-(1-azetidinyl)-9H-purin-9-yl]-2-cyclopentene-1-methanol O-[phenyl(methoxy-L-alaninyl)]phosphoramidate;

(1S,*cis*)-4-[2-Amino-6-(cyclopropylmethylamino)-9H-purin-9-yl]-2-cyclopentene-1-methanol O-[phenyl(methoxy-L-alaninyl)]phosphoramidate;

(1S,*cis*)-4-(2-Amino-6-butoxy-9H-purin-9-yl)-2-cyclopentene-1-methanol O-[phenyl(methoxy-L-alaninyl)]phosphoramidate;
(1S,*cis*)-4-(2-Amino-6-methoxy-9H-purin-9-yl)-2-cyclopentene-1-methanol O-[phenyl(methoxy-L-alaninyl)]phosphoramidate;
(1S,*cis*)-4-[2-Amino-6(propylamino)-9H-purin-9yl]-2-cyclopentene-1-methanol O-[phenyl(methoxy-L-alaninyl)]phosphoramidate;
(1S,*cis*)-4-[2-Amino-6-(isopropylamino)-9H-purin-9-yl]-2-cyclopentene-1-methanol O-(phenyl(methoxy-L-alaninyl)] phosphoramidate;
(1S,*cis*)-4-[2-Amino-6-(allylthio)-9H-purin-9-yl]-2-cyclopentene-1-methanol O-(phenyl(methoxy-L-alaninyl)lphosphoramidate;
(1S,*cis*)-4-(2,6-Diamino-9H-purin-9-yl)-2-cyclopentene-1-methanol O-[phenyl(methoxy-L-alaninyl)]phosphoramidate;
(1S,*cis*)-4-[2-Amino-6-(cyclopropylmethylamino)-9H-purin-9-yl]-2-cyclopentene-1-methanol O-[phenyl(methoxy-α,α-dimethylglycinyl)]phosphoramidate;
(1S,*cis*)-4-[2-Amino-6-(cyclopropylmethylamino)-9H-purin-9-yl]-2-cyclopentene-1-methanol O-[phenyl(benzyloxy-L-alaninyl)]phosphoramidate;
(1S,*cis*)-4-[2-Amino-6-(cyclopropylmethylamino)-9H-purin-9-yl]-2-cyclopentene-1-methanol O-[phenyl(methoxy-L-phenylalaninyl)]phosphoramidate;
and pharmaceutically acceptable derivatives thereof.

Physiologically acceptable salts of the compounds of the present invention include salts of a basic or acidic portion of the molecule. Salts of a basic moiety are formed by organic carboxylic acids such as acetic, lactic, tartaric, malic, isethionic, lactobionic, and succinic acids, organic sulphonic acids, such as methanesulphonic, ethanesulphonic, benzenesulphonic and p-toluenesulphonic acids and inorganic acids, such as hydrochloric, sulphuric, phosphoric and sulphamic acids. Salts of an acidic moiety are formed by an appropriate base , such as an alkali metal (for example, sodium), an alkaline earth (for example, magnesium, calcium), ammonium and ammonium salts.

For therapeutic use, salts of compounds according to the invention will be physiologically acceptable, i.e. they will be salts derived from a physiologically acceptable acid. However, salts of acids which are not physiologically acceptable may also find use, for example, in the preparation or purification of a physiologically acceptable compound. All salts, whether or not derived from a physiologically acceptable acid, are within the scope of the present invention.

Compounds of formula (1) may be made by modifications of the procedures described in Biochem. Biophys. Res. Commun. 225:363-369, 1996.

The present invention futher includes a process for the preparation of a compound according to the invention which comprises reaction of a compound of formula (II)

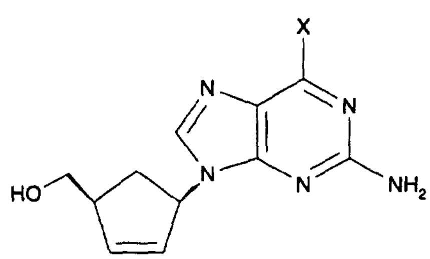

(II)

with a compound of formula (III)

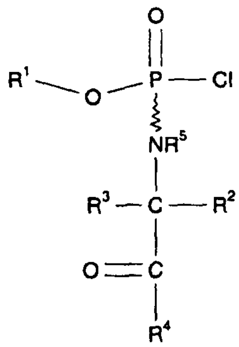

(III)

wherein $R^1$ - $R^5$ are as hereinbefore defined.

The reaction may be carried out in pyridine, pyridine-tetrahydrofuran, or acetonitrile in the presence of t-butyl magnesium chloride (Balzarini et al., *Biochem. Biophys. Res. Comm.* 225:363-369 (1996). The phosphochloridate intermediates may be prepared according to WO 96/29336.

Separation of isomers may be accomplished by methods known in the art, for example, by high-pressure liquid chromatography with chiral columns, particularly using liquid carbon dioxide as the mobile phase, or by crystallization of salts with chiral acids or bases.

Phosphate isomers may be separated with Supercritical Fluid Chromatography using a Chiralpak® AS column, 25% methanol in carbon dioxide as the eluent, flow rate 2 mL/min, temperature 40° C, and pressure 206.8 $10^5$ Pa (3000psi).

One aspect of the invention features the compounds according to the invention for use in medical therapy, particularly for the treatment of retroviral infections, particularly HIV, and hepatitis B virus and hepatitis C virus infections.

A further aspect of the invention features the compounds according to the invention for use in the manufacture of a medicament for the treatment or prophylaxis of viral infections, particularly for the treatment of retroviral infections, particularly HIV, and hepatitis B virus and hepatitis C virus infections.

Examples of retroviral infections which may be treated or prevented in accordance with the invention include human retroviral infections such as human immunodeficiency virus (HIV), HIV-1, HIV-2 and human T-cell lymphotropic virus (HTLV), for example, HTLV-I or HTLV-II infections. The compounds according to the invention are especially useful for the treatment of AIDS and related clinical conditions such as AIDS-related complex (ARC), progressive generalized lymphadenopathy (PGL), AIDS-related neurological conditions, such as multiple sclerosis or tropical paraparesis, anti-HIV antibody-positive and HIV-positive conditions and thrombocytopenic purpura.

The compounds according to the invention are particularly applicable for the treatment of asymptomatic infections or diseases in humans caused by or associated with human retroviruses.

The compounds according to the invention may be stable towards acid-mediated hydrolytic decomposition and thus, advantageous as therapeutic agents for oral administration, because the compounds are likely to withstand the acidic environment of the stomach.

The compounds according to the invention may be employed in combination with other therapeutic agents for the treatment of the above infections or conditions. Other therapeutic agents may include agents that are effective for the treatment of viral infections or associated conditions such as reverse transcriptase inhibitors, for example, zidovudine and abacavir; (1 alpha, 2 beta, 3 alpha)-9-[2,3-bis(hydroxymethyl)cyclobutyl]guanine [(-)BHCG, SQ-34514]; oxetanocin-G (3,4-bis(hydroxymethyl)-2-oxetanosyl]guanine); acyclic nucleosides (e.g. acyclovir, valaciclovir, famciclovir, ganciclovir, penciclovir); acyclic nucleoside phosphonates (e.g. (S)-1-(3-hydroxy-2-phosphonyl-methoxypropyl) cytosine (HPMPC) or PMEA or PMPA; ribonucleotide reductase inhibitors such as hydroxyurea, 2-acetylpyridine 5-[(2-chloroanilino)thiocarbonyl) thiocarbonohydrazone; other 2',3'-dideoxynucleosides such as 2',3'-dideoxycytidine, 2',3'-dideoxyadenosine, 2',3'-dideoxyinosine, 3'-deoxy-2',3'-didehydrothymidine (d4T); protease inhibitors such as saquinavir, indinavir, ritonavir, nelfinavir, amprenavir, tipranavir; oxathiolane nucleoside analogues such as lamivudine, cis- 1-(2-(hydroxymethyl)- 1,3-oxathiolan-5-yl)-5-fluorocytosine (FTC); 3'-deoxy-3'-fluorothymidine, 5-chloro-2',3'-dideoxy-3'-fluorouridine, ribavirin, 9-[4-hydroxy-2-(hydroxymethyl)but-1 -yl]-guanine (H2G); tat inhibitors such as 7-chloro-5-(2-pyrryl)-3H-1,4-benzodiazepin-2-(H)one (Ro5-3335), 7-chloro-1,3-dihydro-5-(1H-pyrrol-2yl)-3H-1,4-benzodiazepin-2-amine (Ro24-7429); interferons such as $\alpha$-interferon; renal excretion inhibitors such as probenecid; nucleoside transport inhibitors such as dipyridamole; pentoxifylline, N-acetylcysteine (NAC), Procysteine, $\alpha$ -trichosanthin, phosphonoformic acid, as well as immunomodulators such as interleukin II or thymosin, granulocyte macrophage colony stimulating factors, erythropoetin, soluble $CD_4$ and genetically engineered derivatives thereof; or non-nucleoside reverse transcriptase inhibitors (NNRTIs) such as nevirapine (BI-RG-587), loviride ($\alpha$ -APA), delavuridine (BHAP),

atevirdine, efavirenz, and DPC 961/963, and phosphonoformic acid, and 1,4-dihydro-2H-3,1-benzoxazin-2-ones NNRTIs such as (-)-6-chloro-4-cyclopropylethynyl-4-trifluoromethyl-1,4-dihydro-2H-3,1-benzoxazin-2-one (L-743,726 or DMP-266) and quinoxaline NNRTIs such as isopropyl (2S)-/-fluoro-3,4-dihydro-2-ethyl-3-oxo-1(2H)-quinoxalinecarboxylate (HBY1293). The component compounds of such combination therapy may be administered simultaneously, in either separate or combined formulations, or at different times, for example, sequentially such that a combined effect is achieved.

The compounds according to the invention, also referred to herein as the active ingredient, may be administered for therapy by any suitable route including oral, rectal, nasal, topical (including buccal and sublingual), vaginal and parenteral (including subcutaneous, intramuscular, intravenous and intradermal). It will be appreciated that the preferred route will vary with the condition and age of the recipient, the nature of the infection and the chosen active ingredient.

The amounts required of the active ingredient will depend upon a number of factors including the severity of the condition to be treated and the identity of the recipient and will ultimately be at the discretion of the attendant physician or veterinarian. In general however, for each of these utilities and indications, a suitable effective dose of a compound of formula (I) will be in the range of 0.01 to 100 mg per kilogram body weight of recipient per day, advantageously in the range of 1 to 70 mg per kilogram body weight per day, preferably in the range of 1 to 50 mg per kilogram body weight per day.

The desired dose is preferably presented as one, two, three or four or more subdoses administered at appropriate intervals throughout the day. These sub-doses may be administered in unit dosage forms, for example, containing about 0.5 to 2000 mg, preferably about 5, 25, 50, 150, 200, or 250 mg of active ingredient per unit dose form.

While it is possible for the active ingredient to be administered alone, it is preferable to present it as a pharmaceutical composition. A further aspect of the present invention features pharmaceutical compositions comprising a compound of formula (I) or a pharmaceutically acceptable derivative thereof and a pharmaceutically acceptable carrier therefor.

The compositions of the present invention comprise at least one active ingredient, as defined above, together with one or more pharmaceutically acceptable carriers thereof and optionally other therapeutic agents. Each carrier must be "acceptable" in the sense of being compatible with the other ingredients of the composition and not deleterious to the recipient thereof. Compositions include those suitable for oral, rectal, nasal, topical (including buccal and sublingual), vaginal or parenteral (including subcutaneous, intramuscular, intravenous and intradermal) administration.

The compositions may conveniently be presented in unit dosage form prepared by any of the methods well known in the art of pharmacy. Such methods include the step of bringing into association the active ingredient with the carrier which constitutes one or more accessory ingredients. In general, the compositions are prepared by uniformly and intimately bringing in to association the active ingredient with liquid carriers or finely divided solid carriers or both, and then, if necessary, shaping the product.

Compositions of the present invention suitable for oral administration may be presented as discrete units such as capsules, cachets, sachets of granules or tablets (such as a swallowable, dispersible or chewable tablet) each containing a predetermined amount of the active ingredient; as a powder or granules; as a solution or a suspension in an aqueous liquid or a non-aqueous liquid; or as an oil-in-water liquid emulsion or a water-in-oil liquid emulsion. The active ingredient may also be presented as a bolus, electuary or paste.

A tablet may be made by compression or moulding optionally with one or more accessory ingredients. Compressed tablets may be prepared by compressing in a suitable machine the active ingredient in a free-flowing form such as a powder or granules, optionally mixed with a binder, lubricant, inert diluent, preservative, surface active or dispersing agent. Moulded tablets may be made by moulding in a suitable machine a mixture of the powdered compound moistened with an inert liquid diluent. The tablets may optionally be coated or scored any may be formulated so as to provide slow or controlled release of the active ingredient therein. Tablets may be enteric coated.

Compositions suitable for topical administration in the mouth include lozenges comprising the active ingredient in a flavored basis, usually sucrose and acacia or tragacanth; pastilles comprising the active ingredient in an inert basis such as gelatin and glycerin, or sucrose end acacia; and mouthwashes comprising the active ingredient in a suitable liquid carrier.

Compositions for rectal administration may be presented as a suppository with a suitable base comprising for example cocoa butter or a salicylate.

Compositions suitable for vaginal administration may be presented as pessaries, tampons, creams, gels, pastes, foams or spray formulations containing in addition to the active ingredient such carriers as are known in the art to be appropriate.

Compositions suitable for parenteral administration include aqueous and non-aqueous isotonic sterile injection solution which may contain anti-oxidants, buffers, bacteriostats and solutes which render the formulation aqueous and non-aqueous sterile suspensions which may include suspending agents and thickening agents. The compositions may be presented in unit-dose or multidose sealed containers, for example, ampoules and vial, and may be stored in

a freeze-dried (lyophilized) condition requiring only the addition of the sterile liquid carrier, for example water for injections, immediately prior to use. Extemporaneous injection solutions and suspensions may be prepared from sterile powders, granules and tablets of the kind previously described.

The active ingredient may also be presented in a composition comprising micrometer- or nanometer-size particles of active ingredient.

Preferred unit dosage compositions are those containing a daily dose or unit daily sub-dose (as herein above recited) or an appropriate fraction thereof, of the active ingredient.

It should be understood that in addition to the ingredients particularly mentioned above the composition of this invention may include other agents conventional in the art having regard to the type of formulation in question, for example those suitable for oral administration may include flavoring agents or taste masking agents.

A further aspect of the invention relates to kits to be used in the treatment of patients suffering from viral infections. These kits include one or more oral dosage of a compound of formula (I) and may include one or more additional therapeutic agents. By way of illustration, a kit of the invention may include one or more tablets, capsules, caplets, gelcaps or liquid formulations containing a compound of formula (I) and one or more tablets, capsules, caplets, gelcaps or liquid formulations containing a compound of formula (1) in dosage amounts within the ranges described above. The kits may include as an insert printed dosing information for the co-administration of the agents.

The following examples are intended for illustration only and are not intended to limit the scope of the invention in any way.

Example 1

(1S,*cis*)-4-[2-Amino-6-(1-azetidinyl)-9H-purin-9-yl]-2-cyclopentene-1-methanol O-[phenyl(methoxy-L-alaninyl) lphosphoramidate

(a) (1S,*cis*)-4-[2-Amino-6-(1-azetidinyl)-9H-purin-9-yl]-2-cyclopentene-1-methanol

A solution of (1S,*cis*)-4-(6-chloro-9H-purin-9-yl)-2-cyclopentene-1-methanol (US Patent 5,917,041, June 1999) (300 mg, 1.13 mmol) and azetidine (1.00 g, 17.5 mmol) in methanol (10 mL) was maintained at 60°C in a sealed Parr bomb for 18h. To the cooled solution was added 1 N sodium hydroxide (1.13 mL). Volatiles were evaporated and the residual solid chromatographed on silica gel. Elution with 6% methanol-chloroform gave title compound as a white solid, after trituration with acetonitrile (280 mg, 87%); m.p. 187-189°C; $[\alpha]_D^{20}$ -31.9 (c 0.25, methanol); $^1$H-NMR(DMSO-$d_6$) δ7 59 (s, 1H), 6.11 (m, 1H), 5.86 (m, 3H), 5.37 (m, 1H), 4.75 (t, J = 52 Hz, 1H), 4.24 (m, 4H), 3.44 (m, 2H), 2.85 (m, 1H), 2.60 (m, 1H), 2.36 (m, 2H), 1.55 (m, 1H).

Anal. Calcd. for $C_{14}H_{18}N_6O$: C, 58.73; H, 6.34; N, 29.35. Found: C, 58.74; H, 6.35; N, 29.43

(b) (1S,*cis*)-4-[2-Amino-6-(1-azetidinyl)-9H-purin-9-yl]-2-cyclopentene-1-methanol O-[phenyl(methoxy-L-alaninyl)] phosphoramidate

Nucleosides were dried by evaporation of portions of dry pyridine. To a stirred solution of dry (1S,*cis*)-4-[2-amino-6-(1-azetidinyl)-9H-purin-9-yl]-2-cyclopentene-1-methanol (600 mg 2.1 mmol) in dry pyridine (5 mL)-tetrahydrofuran (5 mL) under nitrogen was added 1 M tert-butylmagnesium chloride (3.2 mL). After 20 minutes, a solution of phenyl (methoxy-L-alaninyl)phosphorochloridate (prepared as described by C. McGuigan et al. *J. Med. Chem.* **1993**, *36*: 1048-1052) (4.2 mL, 4.2 mmol) in anhydrous tetrahydrofuran was added and the solution was stirred at ambient temperature for 12 h. The resulting mixture was concentrated to a syrup under reduced pressure. This syrup was dissolved in dichloromethane (60 mL) and the organic phase washed with water (2 x 30 mL), then brine (30 mL) and dried (magnesium sulfate), filtered, and concentrated to a foam. This foam was purified by flash column chromatography on silica gel. The title compound was eluted with 5% methanol in chloroform to give, after removal of volatiles, 850 mg (77 %) of a white solid foam; $^1$H-NMR(DMSO-$d_6$) δ 7.58 (s, 1H), 7.34 (m, 2H), 7.16 (m, 3H), 6.05 (m, 5H), 5.4 (m, 1H), 4.24 (m, 4H), 3.95-4.10 (m, 2H), 3.85 (m, 1H), 3.57 (s, 3H), 3.07 (m, 1H), 2.57 (m, 1H), 2.36 (m, 2H), 1.6 (m, 1H), 1.2 (m, 3H); $^{31}$P-NMR(DMSO-$d_6$) 3.786, 3.439.

Anal. Calcd. for $C_{24}H_{30}N_7O_5P \cdot 0.89H_2O$: C, 53.03; H, 5.89; N, 18.04; Found: C, 53.03; H, 5.80; N, 17.94.

Example 2

(1S,*cis*)-4-[2-Amino-6-(cyclopropylmethylamino)-9H-purin-9-yl]-2-cyclopentene-1-methanol O-[phenyl(methoxy-L-alaninyl)]phosphoramidate

(a) (1S,*cis*)-4-[2-Amino-6-(cyclopropylmethylamino)-9H-purin-9-yl]-2-cyclopentene-1-methanol

A solution of (1S,*cis*)-4-(6-chloro-9H-purin-9-yl)-2-cyclopentene-1-methanol (US Patent 5,917,041, June 1999) (4.00 g, 15 mmol) and N-cyclopropyl-N-methylamine *(J. Med Chem.,* **1994**, *37*:3482-3491) (10.7 g, 37.7 mmol) in ethanol (60 mL) was refluxed for 2 h. To the cooled solution was added 1 N sodium hydroxide (15 mL). Volatiles were evaporated and the residual solid chromatographed on silica gel. Elution with 5% methanol-chloroform gave title compound as a white solid foam, after concentration of an ethanol solution (4.30 g, 95%); $[\alpha]_D^{20}$ -49.2 (c 0.23, methanol); $^1$H-NMR(DMSO-$d_6$) δ 7.63 (s, 1H), 6.11 (m, 1H), 5.86 (m, 1H), 5.81 (br s, 2H), 5.41 (m, 1H), 4./4(t, j = 5.2 Hz, 1H), 3.46 (m, 2H), 3.22 (m, 4H), 2.85 (m, 1H), 2.60 (m, 1H), 1.55 (m, 1H), 0.78 (m, 2H), 0.65 (m, 2H).

Anal. Calcd. for $C_{15}H_{20}N_6O \cdot 0.25\ C_2H_5OH \cdot 0.59H_2O$; C, 57.73; H,: 7.09; N, 26.06. Found: C, 57.73; H, 6.99; N, 26.04.

(b) (1S,*cis*)-4-[2-Amino-6-(cyclopropylmethylamino)-9H-purin-9-yl]-2-cyclopentene-1-methanol O-[phenyl(methoxy-L-alaninyl)]phosphoramidate

The title compound was prepared by the method of Example 1, part b, from (1S,*cis*)-4-[2-amino-6-(cyclopropylmethylamino)-9H-purin-9-yl]-2-cyclopentene-1-methanol (part a of this example, 1.20 g, 3.70 mmol) and isolated as a white solid foam (1.30 g. 65%), after elution from a silica gel column with 5% methanol-chloroform; $^1$H-NMR (DMSO-$d_6$) δ 7.61 (s, 1H). 7.34 (m, 2H), 7.16 (m, 3H), 5.8-6.1 (m, 5H), 5.45 (m, 1H), 3.95-4.10 (m, 2H), 3.85 (m, 1H), 3.57 (s, 3H), 3.25 (m, 4H), 3.09 (m, 1H), 2.57 (m, 1H), 1.6 (m, 1H), 1.2 (m, 3H), 0.83 (m, 2H), 0.67 (m, 2H); $^{31}$P-NMR (DMSO-$d_6$) 3.766, 3.424.

Anal. Calcd. for $C_{25}H_{32}N_7O_5P \cdot 0.14\ CH_3CN \cdot 0.09\ H_2O$: C, 55.32; H, 5.99; N, 18.22. Found: C, 55.31; H, 6.01; N, 18.22.

Example 3

(1S,*cis*)-4-(2-Amino-6-butoxy-9H-purin-9-yl)-2-cyclopentene-1-methanol O-[phenyl(methoxy-L-alaninyl)] phosphoramidate

(a) (1S,*cis*)-4-(2-Amino-6-butoxy-9H-purin-9-yl)-2-cyclopentene-1-methanol

To a solution of (1S,*cis*)-4-(6-chloro-9H-purin-9-yl)-2-cyclopentene-1-methanol (US Patent 5,917,041, June 1999) (4.00 g, 15 mmol) in dry n-butanol (50 mL) was added sodium hydride (60% in mineral oil, 723 mg, 18 mmol). The resulting mixture was maintained at 55°C for 3 h. Volatiles were evaporated and the residual solid chromatographed on silica gel. Elution with 5% methanol-chloroform gave title compound as a white powder, after solidification from methanol-acetonitrile (3.70 g, 81%); $[\alpha]_D^{20}$ -77.5 (c 0.24, methanol); $^1$H-NMR(DMSO-$d_6$) δ 7.75 (s, 1H), 6.36 (s, 2H), 6.11 (m, 1H), 5.87 (m, 1H), 5.42 (m, 1H), 4.73 (t, J = 5.2 Hz, 1H), 4.40 (t, J = 6.7 Hz, 2H), 3.44 (m, 2H), 2.88(m, 1H), 2.00(m, 1H), 1,73 (m, 2H), 1.60 (m, 1H), 1.41 (m, 2H), 0.94 (t, J = 7.3 Hz, 3H).

Anal. Calcd. for $C_{15}H_{21}N_5O_2 \cdot 0.05CH_3CN \cdot 0.47H_2O$: C, 57.78; H, 7.09; N, 22.54. Found: C, 57.77; H, 6.99; N, 22.53.

(b) (1S,*cis*)-4-(2-Amino-6-butoxy-9H-purin-9-yl)-2-cyclopentene-1-methanol O-[phenyl(methoxy-L-alaninyl)] phosphoramidate

The title compound was prepared by the method of Example 1, part b, from (1S,*cis*)-4-(2-amino-6-butoxy-9H-purin-9-yl)-2-cyclopentene-1-methanol (part a of this example, 1.00 g, 3.30 mmol) and isolated as a white solid foam (1.30 g, 75%), after elution from a silica gel column with 5% methanol-chloroform; $^1$H-NMR(DMSO-$d_6$) δ 7.73 and 7.72 (both s, 1H), 7.34 (m, 2H), 7.16 (m, 3H), 6.38 (s, 2H), 6.05 (m, 3H), 5.46 (m, 1H), 4.40 (t, J = 6.5 Hz, 2H), 3.95-4.15 (m, 2H), 3.85 (m, 1H), 3.57 (s, 3H), 3.07 (m, 1H), 2.65 (m, 1H), 1.7 (m, 3H), 1.4 (m, 2H), 1.2 (m, 3H), 0.93 (t, J = 7.4 Hz, 3H); $^{31}$P-NMR(DMSO-$d_6$): 3.801, 3.459.

Anal. Calcd. for $C_{25}H_{33}N_6O_6P \cdot 0.06CH_3CN \cdot 0.02H_2O$: C, 55.12; H, 6.12; N, 15.51. Found: C, 55.12; H, 6.12; N, 15.51.

Example 4

(1S,*cis*)-4-(2-Amino-6-methoxy-9H-purin-9-yl)-2-cyclopentene-1-methanol O-[phenyl(methoxy-L-alaninyl))
phosphoramidate

(a) (1S,*cis*)-4-(2-Amino-6-methoxy-9H-purin-9-yl)-2-cyclopentene-1-methanol

To a solution of (1 S,cis)-4-(6-chloro-9H-purin-9-yl)-2-cyclopentene-1-methanol (US Patent 5,917,041, June 1999) (4.00 g, 15 mmol) in dry methanol (60 mL) was added sodium hydride (60% in mineral oil, 1.2 g, 30 mmol). The resulting mixture was refluxed for 2 h. The solution was cooled (ice bath) while 1 N hydrochloric acid (15 mL) was added. Volatiles were evaporated and the residual solid chromatographed on silica gel. Elution with 5% methanol-chloroform gave title compound as a white solid foam, after evaporation of an ethanol solution (3.80 g, 97%); $[\alpha]_D^{20}$ -102.5 (c 0.57, methanol, $^1$H-NMR(DMSO-$d_6$) δ 7.77 (s, 1H), 6.42 (s, 2H), 6.11 (m, 1H), 5.88 (m, 1H), 5.43 (m, 1H), 4.74 (t, J = 5.2 Hz, 1H), 4.00 (s, 3H), 3.44 (m, 2H), 2.86 (m, 1H), 2.60 (m, 1H), 1.60 (m, 1H).

Anal. Calcd. for $C_{12}H_{15}N_5O_2 \cdot 0.29C_2H_5OH \cdot 0.21H_2O$: C, 54.27; H, 6.21; N, 25.15. Found: C, 54.25; H, 6.26; N, 25.16.

(b) (1S,*cis*)-4-(2-Amino-6-methoxy-9H-purin-9-yl)-2-cyclopentene-1-methanol O-[phenyl(methoxy-L-alaninyl)]
phosphoramidate

The title compound was prepared by the method of Example 1, part b, from (1S,*cis*)-4-(2-amino-6-methoxy-9H-purin-9-yl])-2-cyclopentene-1-methano) (part a of this example, 1.00 g, 3.80 mmol) and isolated as a white solid foam (1.30 g, 68%), after elution from a silica gel column with 3% methanol-chloroform; $^1$H-NMR(DMSO-$d_6$) δ 7.74 and 7.73 (both s, 1H), 7.34 (m, 2H), 7.16 (m, 3H), 6.40 (br s, 2H), 5.95-6.05 (m, 6H), 5.46 (m, 1H), 3.9-4.1 (m, 2H), 3.95 (s, 3H), 3.85 (m, 1H), 3.57 (s, 3H). 3.1 (m, 1H), 2.65 (m, 1H), 1.65 (m, 1H), 1.2 (m, 3H); $^{31}$P-NMR(DMSO-$d_6$) 3.786, 3.439.

Anal. Calcd. for $C_{22}H_{27}N_6O_6P \cdot 0.21H_2O$: C, 52.20; H, 5.46; N, 16.60. Found: C, 52.20; H, 5.50; N, 16.52.

Example 5

(1S,*cis*)-4-[2-Amino-6-(propylamino)-9H-purin-9-yl]-2-cyclopentene-1-methanol O-[phenyl(methoxy-L-alaninyl)]
phosphoramidate

(a) (1S,*cis*)-4-[2-Amino-6-(propylamino)-9H-purin-9-yl]-2-cyclopentene-1-methanol

A solution of (1S,*cis*)-4-(6-chloro-9H-purin-9-yl)-2-cyclopentene-1-methanol (US Patent 5,917,041, June 1999) (1.00 g, 3.77 mmol) and propylamine (3.1 mL, 38 mmol) in ethanol (16 mL) was refluxed for 3 h. To the cooled solution was added 5 N sodium hydroxide (0.76 mL). Volatiles were evaporated and the residual solid chromatographed on silica gel. Elution with 5% methanol-chloroform gave title compound as a white powder, after solidification from methanol-acetonitrile (1.00 g, 92%); $[\alpha]_D^{20}$ --57.1 (c 0.30, methanol); m.p. 146-148°C; $^1$H-NMR(DMSO-$d_6$) δ 7.59 (s, 1H), 7.16 (br s, 2H), 6.10(m, 1H), 5.86 (m,1H), 5.78 (s, 2H), 5.38 (m, 1H), 4.73 (t, J = 5.2 Hz, 1H), 3.45 (m, 4H), 2.86 (m, 1H), 2.60 (m, 1H), 1.57 (m, 4H), 0.87 (t, J = 7.4 Hz, 3H).

Anal. Calcd. for $C_{14}H_{20}N_6O$: C: 58.31; H, 6.99; N, 29.15. Found: C, 58.21; H, 7.05; N, 29.16.

(b) (1S,*cis*)-4-[2-Amino-6-(propylamino)-9H-purin-9-yl]-2-cyclopentene-1-methanol O-[phenyl(methoxy-L-alaninyl)]
phosphoramidate

The title compound was prepared by the method of Example 1, part b, from (1S,*cis*)-4-[2-amino-6-(propylamino)-9H-purin-9-yl]-2-cyclopentene-1-methanol (part a of this example, 1.00 g, 3.50 mmol) and isolated as a white solid foam (1.20 g, 64%), after elution from a silica gel column with 5% methanol-chloroform; $^1$H-NMR(DMSO-$d_6$) δ7.57 (s, 1H), 7.34 (m, 2H), 7.16 (m, 4H), 6.05 (m, 3H), 5.79 (br s, 2H), 5.41 (m, 1H), 3.95-4.15 (m, 2H), 3.85 (m, 1H), 3.57 (s, 3H), 3.38 (m, 2H), 3.07(m, 1H), 2.65 (m, 1H,m), 1.6 (m, 3H), 1.2 (m, 3H), 0.87 (t, J=7.2 Hz, 3H); $^{31}$P-NMR(DMSO-$d_6$) 3.781, 3.455.

Anal. Calcd. for $C_{24}H_{32}N_7O_5P$ $0.58H_2O$: C, 53.38; H, 6.19; N, 18.16. Found: C, 53.38; H, 6.04; N, 18.04.

Example 6

(1S,*cis*)-4-[2-Amino-6-(isopropylamino)-9H-purin-9-yl]-2-cyclopentene-1-methanol O-[phenyl(methoxy-L-alaninyl)] phosphoramidate

(a) (1S,*cis*)-4-[2-Amino-6-(isopropylamino)-9H-purin-9-yl]-2-cyclopentene-1-methanol

A solution of (1S,*cis*)-4-(6-chloro-9H-purin-9-yl)-2-cyclopentene-1-methanol (US Patent 5,917,041, June 1999) (1.00 g, 3.77 mmol) and isopropylamine (3.2 mL, 38 mmol) in ethanol (16 mL) was refluxed for 4 h. To the cooled solution was added 5 N sodium hydroxide (0.76 mL). Volatiles were evaporated and the residual solid chromatographed on silica gel. Elution with 5% methanol-chloroform gave title compound as a white powder, after solidification from ethyl acetate - hexanes (1.00 g, 85%); $[\alpha]_D^{20}$ -50.6 (c 0.37, methanol); m.p. 87-90°C; $^1$H-NMR(DMSO-$d_6$) δ 7.59 (s, 1H), 6.88 (m, 1H), 6.10 (m, 4H), 5.85 (m, 1H), 5.77 (br s, 2H), 5.38 (m, 1H), 4.75 (t, J = 5.2 Hz, 1H), 4.43 (m, 1H), 3.44 (m, 2H), 2.85(m, 1H), 2.60(m, 1H), 1.57 (m, 3H), 1.17 (d. J = 6.4 Hz, 6H).

Anal. Calcd. for $C_{14}H_{20}N_6O$ 0.0.07 EtOAc 0.19 $H_2O$: C, 57.57; H, 7.08; N, 28.21. Found: C, 57.62; H, 7.08; N, 28.22.

(b) (1S,*cis*)-4-[2-Amino-6-(isopropylamino)-9H-purin-9-yl]-2-cyclopentene-1-methanol O-[phenyl(methoxy-L-alaninyl)] phosphoramidate

The title compound was prepared by the method of Example 1, part b, from (1S,*cis*)-4-[2-amino-6-(isopropylamino)-9H-purin-9-yl]-2-cyclopentene-1-methanol (part a of this example, 1.00 g. 3.10 mmol) and isolated as a white solid foam (1.00 g, 61%), after elution from a silica gel column with 5% methanol-chloroform; $^1$H-NMR(DMSO-$d_6$) δ 7.57 (s, 1H), 7.34 (m, 2H), 7.16 (m, 3H), 6.90 (m, 1H), 6.05 (m, 3H), 5.79 (br s, 2H), 5.41 (m, 1H), 4.40 (m, 1H), 3.95-4.10 (m, 2H), 3.85 (m, 1H), 3.57 (s, 3H), 3.07 (m, 1H), 2.57 (m, 1H), 1.6 (m, 1H), 1.2 (m, 9H); $^{31}$P-NMR(DMSO-$d_6$) 3.771, 3.444.

Anal. Calcd. for $C_{24}H_{32}N_7O_5P$ 0.38$H_2O$: C: 53.74; H, 6.16; N, 18.28. Found: C, 53.74; H, 6.17; N, 18.16.

Example 7

(1S,*cis*)-4-[2-Amino-6-(allylthio)-9H-purin-9-yl]-2-cyclopentene-1-methanol O-[phenyl(methoxy-L-alaninyl)] phosphoramidate

(a) (1S,*cis*)-4-(2-amino-1,6-dihydro-6-thioxo-9H-purin-9-yl)-2-cyclopentene-1-methanol

(1S,*cis*)-4-(6-Chloro-9H-purin-9-yl)-2-cyclopentene-1-methanol (US Patent 5,917,041, June 1999) (481 mg, 1.81 mmol) and thiourea (151 mg, 1.99 mmol) were refluxed in n-propanol (6 mL) for 1.5 h. The resulting mixture was cooled (ice bath) and the precipitate collected after neutralization 1N sodium hydroxide. Recrystallization from ethanol-water gave title compound as off-white powder (300 mg, 63%); m.p. >250°C; $[\alpha]_D^{20}$ -29.1 (c 0.24, methanol).

Anal. Calcd. for $C_{11}H_{13}N_5OS$: C, 50.18; H, 4.98; N, 26.60; S, 12.18. Found: C, 50.20; H, 5.00; N, 26.54; S, 12.22.

(b) (1S,*cis*)-4-[2-Amino-6-(allylthio)-9H-purin-9-yl]-2-cyclopentene-1-methanol

Allyl chloride (0.43 mL) was added to a solution of (1S,*cis*)-4-(2-amino-1,6-dihydro-6-thioxo-9H-purin-9-yl)-2-cyclopentene-1-methanol (part a of this example, 210 mg, 0.80 mmol) in dioxane (2 mL) and 1N sodium hydroxide (0.80 mL). The solution was stirred for 2 h. Water (10 mL)was added and the crude product was extracted into chloroform (3x10 mL). The chloroform extracts were dried ($MgSO_4$), volatiles evaporated, and the residual gum chromatographed on silica gel. Title compound eluted with 5% methanol-chloroform as an off-white solid foam (212 mg, 88%), after evaporation of an ethyl acetate solution; $[\alpha]_D^{20}$ -53.5 (c 0.245, methanol).

Anal. Calcd. for $C_{14}H_{17}N_5OS$ 0.15 EtOAc 0.25 $H_2O$: C, 54.61; H, 5.87; N, 21.81; S, 9.99. Found: C, 54.52; H, 5.92; N, 21.89; S, 9.93.

(c) (1S,*cis*)-4-[2-Amino-6-(allylthio)-9H-purin-9-yl]-2-cyclopentene-1-methanol O-[phenyl(methoxy-L-alaninyl)] phosphoramidate

The title compound was prepared by the method of Example 1, part b, from (1S,*cis*)-4-[2-amino-6-(allylthio)-9H-purin-9-yl]-2-cyclopentene-1-methanol (part b of this example, 700 mg, 2.30 mmol) and isolated as a white solid (1.10 g, 88%). after elution from a silica gel column with 3% methanol-chloroform and evaporation of an ethanol solution;

MS ($ES^+$) 545 (M+1); $^{31}$P-NMR(DMSO-$d_6$) δ 3.816, 3.464.

Anal. Calcd. for $C_{24}mH_{29}N_6O_5PS$ 0.38 EtOH 0.45 $H_2O$: C: 52.16; H, 5.69; N, 14.74. Found: C, 52.16; H, 5.59; N, 14.74.

Example 8

(1S,*cis*)-4-(2,6-Diamino-9H-purin-9-yl)-2-cyclopentene-1-methanol O-[phenyl(methoxy-L-alaninyl)]phosphoramidate

(a) (1S,*cis*)-4-(2,6-Diamino-9H-purin-9-yl)-2-cyclopentene-1-methanol

(1S,*cis*)-4-(6-Chloro-9H-purin-9-yl)-2-cyclopentene-1-methanol (US Patent 5,917,041, June 1999) (274 mg, 1.00 mmol) and ammonia (1.40 ml) were stirred in a sealed Parr bomb at 60°C for 3 days. After dilution with -78°C methanol (40 mL), the ammonia was allowed to evaporate and 1 N sodium hydroxide (1.00 mL) was added. Volatiles were evaporated and the residual solid chromatographed on silica gel. Elution with 15% methanol-chloroform gave the title compound as a white powder (208 mg, 85%), after slurrying in acetonitrile; m.p. 174-176°C; $[\alpha]_D^{20}$ -75.7 (c 0.25, methanol).

Anal. Calcd. for $C_{11}H_{14}N_6O$: C, 53.65; H, 5.73; N, 34.13. Found: C, 53.54; H, 5.73; N, 34.20.

(b) (1S,*cis*)-4-(2,6-Diamino-9H-purin-9-yl)-2-cyclopentene-1-methanol O-[phenyl(methoxy-L-alaninyl)] phosphoramidate

The title compound was prepared by the method of Example 1, part b, from (1S,*cis*)-4-(2,6-diamino-9H-purin-9-yl)-2-cyclopentene-1-methanol (part a of this example, 1.00 g, 4.06 mmol) and isolated as a white solid foam (730 mg, 37%), after elution from a silica gel column with 7.5% methanol-chloroform; $^1$H-NMR(DMSO-$d_6$) δ 7.59 and 7.58 (both s, 1H), 7.34 (m, 2H), 7.17 (m, 3H), 6.73 (br s, 2H), 5.9-6.1 (m, 3H), 5.83 (br s, 2H), 5.41 (m, 1H), 3.95-4.10 (m, 2H), 3.85 (m, 1H), 3.58 (s, 3H), 3.09 (m, 1H), 2.65 (m. 1H), 1.64 (m, 1H), 1.21 (m, 3H);$^{31}$P-NMR(DMSO-$d_6$) 3.796, 3.464.

Anal. Calcd. for $C_{21}H_{26}N_7O_5P$ 0.06 $CH_3CN$ 0.44 $H_2O$: C: 50.95; H, 5.48; N, 19.86. Found: C, 50.95; H, 5.41; N, 19.86.

Example 9

(1S,*cis*)-4-[2-Amino-6-(cyclopropylmethylamino)-9H-purin-9-yl]-2-cyclopentene-1-methanol O-[phenyl(methoxy-α,α-dimethylglycinyl)]phosphoramidate

By the method of part b of Example 1, (1S,*cis*)-4-[2-amino-6-(cyclopropylmethylamino)-9H-purin-9-yl]-2-cyclopentene-1-methanol (part a of Example 2, 200 mg, 0.67 mmol) was treated with phenyl(methoxy-α,α-dimethylglycinyl)phosphorochloridate (1.7 mL of a 1 M solution in tetrahydrofuran, 1.7 mmoles; prepared as described by C. McGuigan et al. *Antiviral Research* **1997**, *35*:195-204). Title compound was isolated as a pale yellow solid foam (290 mg, 78%), after elution from a silica gel column with 3% methanol-chloroform; $^1$H-NMR(DMSO-$d_6$) δ 7.62, 7.61 (both s, 1H), 7.34 (m, 2H), 7.18 (m, 3H), 6.06 (m, 1H), 5.95 (m, 1H), 583 (m, 3H), 5.44 (m, 1H), 4.06 (m, 2H), 3.55 (s, 3H), 3.25 (m, 4H), 3.09 (m, 1H), 2.6 (m, 1H), 1.6 (m, 1H), 1.2 (m, 6H), 0.79 (m, 2H), 0.67 (m, 2H); $^{31}$P-NMR(DMSO-$d_6$) 2.423, 2.388.

Anal. Calcd. for $C_{26}H_{34}N_7O_5P$ 0.02 $CH_3CN$ 0.12 $H_2O$: C, 56.00; H, 6.19; N, 17.60. Found: C, 56.00; H, 6.26; N, 17.60.

Example 10

(1S,*cis*)-4-[2-Amino-6-(cyclopropylmethylamino)-9H-purin-9-yl]-2-cyclopentene-1-methanol O-[phenyl(benzyloxy-L-alaninyl)]phosphoramidate

By the method of part b of Example 1,(1S,*cis* -4-[2-amino-6-(cyclopropylmethylamino)-9H-purin-9-yl]-2-cyclopentene-1-methanol (part a of Example 2, 200 mg, 0.67 mmol) was treated with phenyl(benzyloxy-L-alaninyl)phosphorochloridate (1.7 mL of a 1 M solution in tetrahydrofuran, 1.7 mmoles; prepared as described by C. McGuigan et al. *Antiviral Research* **1997**, *35*:195-204). Title compound was isolated as a pale yellow solid foam (160 mg, 39%). after elution from a silica gel column with 3% methanol-chloroform; $^1$H-NMR(DMSO-$d_6$) δ 7.60 (s, 1H), 7.33 (m, 7H), 7.18 (m, 3H), 5.8-6.1 (m, 5H), 5.44 (m, 1H), 5.08 (m, 2H), 3.8-4.05 (m, 3H), 3.24 (s and m, 4H), 3.01 (m, 1H), 2.62(m, 1H), 1.55 (m, 1H), 1.42 (m, 3H), 0.79 (m, 2H), 0.67 (m, 2H); $^{31}$P-NMR(DMSO-$d_6$) 4.047, 3.610.

Anal. Calcd. for $C_{31}H_{36}N_7O_5P$ 0.15 $CH_3CN$ 0.47 $H_2O$: C, 59.46; H, 5.96; N, 15.84. Found: C, 59.45; H, 5.95; N,

15.84.

Example 11

(1S,*cis*)-4-[2-Amino-6-(cyclopropylmethylamino)-9H-purin-9-yl]-2-cyclopentene-1-methanol O-[phenyl(methoxy-L-phenylalaninyl)]phosphoramidate

By the method of part b of Example 1, (1S,*cis*)-4-[2-amino-6-(cyclopropylmethylamino)-9H-purin-9-yl]-2-cyclopentene-1-methanol (part a of Example 2, 200 mg, 0.67 mmol) was treated with phenyl(methoxy-L-phenylalaninyl) phosphorochloridate (1.7 mL of a 1 M solution in tetrahydrofuran, 1.7 mmoles; prepared as described by C. McGuigan et al. *Antiviral Research* **1997**, *35*:195 204). Title compound was isolated as a pale yellow solid foam (170 mg, 41%), after elution from a silica gel column with 3% methanol-chloroform; $^1$H-NMR(DMSO-$d_6$) δ7.58 and 7.56 (both s, 1H), 6.95-7.3 (m, 10H), 5.8-6.2 (m, 5H), 5.405 (m, 1H), 3.7-4.0 (m, 2H), 3.53 (s and m, 4H), 3.25 (s and m, 4H), 2.9 (m, 1H), 2.45-2.8 (m, 5H), 1.55 (m, 1H), 0.79 (m, 2H), 0.67 (m, 2H); $^{31}$P-NMR(DMSO-$d_6$) 4.047, 3.610.

Anal. Calcd. for $C_{31}H_{36}N_7O_5P$ 0.25 $CH_3CN$ 0.40 $H_2O$: C, 59.57; H, 5.96; N, 15.99. Found: C, 59.57; H, 5.93; N, 15.99.

Example 12

Anti-HIV Activity

Compounds were tested for anti-HIV activity in $MT_4$ cells according to the method described by Averett, D. R., J. *Virol. Methods,* 23, 1989, 263-276. Activity of the compounds against HIV was in the range $IC_{50}$ 0.010 μM - 2.5 μM.

Example 13

Anti-Hepatitis B Virus Activity

Compounds were tested for anti- hepatitis B Virus activity according to the method described by Jansen, R. et al., *Antimicrobial Agents and Chemotherapy* Vol. 37, No. 3, pp. 441-447, 1993. Anti-hepatitis B activity of the compounds was in the range $IC_{50}$ 0.020 μM - 4.0 μM.

Example 14: Tablet Formulation

The following formulations A, B and C are prepared by wet granulation of the ingredients with a solution of povidone, followed by addition of magnesium stearate and compression.

Formulation A

| | mg/tablet |
|---|---|
| Active Ingredient | 250 |
| Lactose B.P. | 210 |
| Povidone B.P. | 15 |
| Sodium Starch Glycollate | 20 |
| Magnesium Stearate | 5 |
| | 500 |

Formulation B

| | mg/tablet |
|---|---|
| Active Ingredient | 250 |

(continued)

| | mg/tablet |
|---|---|
| Lactose B.P. | 150 |
| Avicel PH 101 | 60 |
| Povidone B.P. | 15 |
| Sodium Starch Glycollate | 20 |
| Magnesium Stearate | 5 |
| | 500 |

Formulation C

| | mg/tablet |
|---|---|
| Active ingredient | 250 |
| Lactose B.P. | 200 |
| Starch | 50 |
| Povidone | 5 |
| Magnesium Stearate | 4 |
| | 359 |

The following formulations, D and E, are prepared by direct compression of the admixed ingredients. The lactose in formulation E is of the direct compression type (Dairy Crest-"Zeparox").

Formulation D

| | mg/tablet |
|---|---|
| Active ingredient | 250 |
| Pregelatinized Starch NF15 | 150 |
| | 400 |

Formulation E

| | mg/tablet |
|---|---|
| Active Ingredient | 250 |
| Lactose B.P. | 150 |
| Avicel | 100 |
| | 500 |

Formulation F (Controlled Release Formulation)

The formulation is prepared by wet granulation of the ingredients with a solution of povidone followed by the addition of magnesium stearate and compression.

| | mg/tablet |
|---|---|
| Active ingredient | 500 |
| Hydroxypropylmethylcellulose | 112 |

(continued)

| | mg/tablet |
|---|---|
| (Methocel K4M Premium) | |
| lactose B.P. | 53 |
| Povidone B.P. | 28 |
| Magnesium Stearate | 7 |
| | 700 |

Drug release takes place over a period of about 6-8 hours and is complete after 12 hours.

Example 15: Capsule Formulations

Formulation A

A capsule formulation is prepared by admixing the ingredients of formulation D in Example 6 above and filling into a two-part hard gelatin capsule. Formulation B (infra) is prepared in a similar manner.

Formulation B

| | mg/capsule |
|---|---|
| Active Ingredient | 250 |
| Lactose B.P. | 143 |
| Sodium Starch Glycollate | 25 |
| Magnesium Stearate | 2 |
| | 420 |

Formulation C

| | mg/capsule |
|---|---|
| Active ingredient | 250 |
| Macrogel 4000 B.P. | 350 |
| | 600 |

Capsules of formulation C are prepared by melting the Macrogel 4000 B.P., dispersing the active ingredient in the melt and filling the melt into a two-part hard gelatin capsule.

Formulation D

| | mg/capsule |
|---|---|
| Active Ingredient | 250 |
| Lecithin | 100 |
| Arachis Oil | 100 |
| | 450 |

Capsules of formulation D are prepared by dispersing the active ingredient in the lecithin and arachis oil and filling the dispersion into soft, elastic gelatin capsules.

Formulation E

| | mg/capsule |
|---|---|
| Active Ingredient | 150.0 |
| Vitamin E TPGS | 400.0 |
| Polyethylene Glycol 400 NF | 200.5 |
| Propylene Glycol USP | 39.5 |

Four (4) kilograms (kg) of Vitamin E TPGS (obtained from Eastman Chemical Co.) was heated at 50°C until liquefied. To the liquified Vitamin E TPGS, 2.005 kg of polyethylene glycol 400 (PEG400) (low aldehyde, <10 ppm, obtained from Union Carbide or Dow Chemical Co.) heated to 50°C was added and mixed until a homogeneous solution was formed. The resultant solution was heated to 65°C. 1.5 kg of active ingredient was dissolved in the liquefied solution of Vitamin E TPGS and PEG 400. 0.395 kg of propylene glycol at room temperature was added and mixed until a homogenous solution was formed. The solution was cooled to 28-35°C. The solution was then de-gassed. The mixture was preferably encapsulated at 28-35°C at a fill weight equivalent to 150 mg of volatiles-free compound, into Size 12 oblong, white opaque soft gelatin capsules using a capsule filling machine. The capsule shells were dried to a constant fill moisture of 3-6% water and a shell hardness of 7-10 Newtons, and placed in a suitable container.

Formulation F (Controlled Release Capsule)

The following controlled release capsule formulation is prepared by extruding ingredients a, b, and c using an extruder, followed by spheronization of the extrudate and drying. The dried pellets are then coated with release-controlling membrane (d) and filled into a two-piece, hard gelatin capsule.

| | | mg/capsule |
|---|---|---|
| (a) | Active ingredient | 250 |
| (b) | Microcrystalline Cellulose | 125 |
| (c) | Lactose B.P. | 125 |
| (d) | Ethyl Cellulose | 13 |
| | | 513 |

Example 16: Injectable Formulation

Formulation A

| | mg |
|---|---|
| Active Ingredient | 200 |
| Hydrochloric Acid Solution 0.1M or | |
| Sodium Hydroxide Solution 0.1M q.s. to pH | 4.0 to 7.0 |
| Sterile water q.s. to | 10 ml |

The active ingredient is dissolved in most of the water (35° - 40° C) and the pH adjusted to between 4.0 and 7.0 with the hydrochloric acid or the sodium hydroxide as appropriate. The batch is then made up to volume with water and filtered through a sterile micropore filter into sterile 10 ml amber glass vial (type 1) and sealed with sterile closures and overseals.

Formulation B

| | |
|---|---|
| Active Ingredient | 125 mg |
| Sterile, Pyrogen-free, pH 7 Phosphate Buffer, q.s. to | 25 ml |

Example 17: Intramuscular Injection

| | |
|---|---|
| Active Ingredient | 200 mg |
| Benzyl Alcohol | 0.10 g |
| Glycofurol 75 | 1.45 g |
| Water for injection q.s. to | 3.00 ml |

The active ingredient is dissolved in the glycofurol. The benzyl alcohol is then added and dissolved, and water added to 3 ml. The mixture is then filtered through a sterile micropore filter and sealed in sterile 3 ml amber glass vials (type 1).

Example 18: Syrup

| | |
|---|---|
| Active Ingredient | 250 mg |
| Sorbitol Solution | 1.50 g |
| Glycerol | 2.00 g |
| Sodium Benzoate | 0.005 g |
| Flavor, Peach 17.42.3169 | 0.0125 ml |
| Purified Water q.s. to | 5.00 ml |

The active ingredient is dissolved in a mixture of the glycerol and most of the purified water. An aqueous solution of the sodium benzoate is then added to the solution, followed by addition of the sorbital solution and finally the flavor. The volume is made up with purified water and mixed well.

Example 19: Suppository

| | mg/capsule suppository |
|---|---|
| Active Ingredient | 250 |
| Hard Fat, B.P. (Witepsol H15-Dynamit Nobel) | 1770 |
| | 2020 |

One-fifth of the Witepsol H15 is melted in a steam-jacketed pan at 45°C maximum. The active ingredient is sifted through a 200μm sieve and added to the molten base with mixing, using a Silverson fitted with a cutting head, until a smooth dispersion is achieved. Maintaining the mixture at 45° C, the remaining Witepsol H15 is added to the suspension and stirred to ensure a homogenous mix. The entire suspension is passed through a 250μm stainless steel screen and, with continuous stirring, is allowed to cool to 45° C. At a temperature of 38° C to 40° C, 2.02 g of the mixture is filled into suitable, 2 ml plastic molds. The suppositories are allowed to cool to room temperature.

Example 20: Pessaries

| | mg/pessary |
|---|---|
| Active Ingredient | 250 |
| Anhydrate Dextrose | 380 |
| Potato Starch | 363 |
| Magnesium Stearate | 7 |
| | 1000 |

The above ingredients are mixed directly.

Example 21: Acid Stability

Compounds were tested for their stability towards acid-mediated hydrolytic decomposition employing a test designed to simulate stomach conditions. Each compound was incubated at an initial concentration of 0.3 mg/mL in dilute hydrochloric acid at pH 1 for 24 hours at 25°C. HPLC was run immediately for t=0 and at intervals up to approximately 24 hours. The results for compounds of Example 2, part b, Example 9, and for comparative phosphoramidates of 2',3'-dideoxy-adenosine (Compound 1093) and 2',3'-didehydro-2',3'-dideoxy-adenosine (Compound 1001), described in W096/29336A, are given in the table below.

| Compound | Time (hr) | Compound left (%) |
|---|---|---|
| 1093 | 0 | 100 |
| | 13 | 0 |
| 1001 | 0 | 0 |
| | 17 | 0 |
| Example 2b | 0 | 100 |
| | 13 | 83 |
| | 24 | 74 |
| Example 9 | 0 | 100 |
| | 13 | 96 |
| | 24 | 94 |

Compound 1001 disappeared immediately (<1 minute). Compound 1093 degraded after less than 13 hours. The majority of the compounds of Example 2b and 9 remained intact after 24 hours.

## Claims

1. A compound of formula (1)

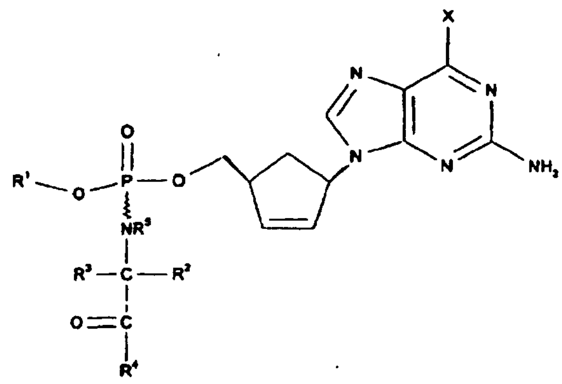

(I)

wherein:

$R^1$ is hydrogen; $C_{6-14}$aryl; or heteroaryl, optionally substituted with one or more substituents selected from the group consisting of $C_{1-6}$alkoxy, nitro, halogen, amino, hydroxy, carboxylate and esters thereof, carboxyalkyl, -CONHR$^6$, and -CONR$^6$R$^7$, wherein $R^6$ and $R^7$, which may be the same or different, are independently selected from $C_{1-8}$alkyl, $C_{1-8}$alkylaryl or $C_{6-14}$aryl;

$R^2$ and $R^3$ are independently selected from hydrogen or $C_{1-8}$alkyl, $C_{3-8}$cycloalkyl, $C_{2-8}$alkenyl, $C_{5-8}$cycloalkenyl, $C_{6-14}$aryl, or aralkyl wherein each $C_{1-8}$alkyl, $C_{3-8}$cycloalkyl, $C_{2-8}$alkenyl, $C_{5-8}$cycloalkenyl, $C_{6-14}$aryl or aralkyl may be optionally substituted with one or more substituents selected from the group consisting of $C_{1-8}$alkyl, halo, hydroxy, alkoxy, amino, aminoalkyl, aminodialkyl, -SH, thioalkyl, carboxylate and esters thereof, carboxyalkyl, -CONHR$^6$, and -CONR$^6$R$^7$, wherein $R^6$ and $R^7$, which may be the same or different, are independently selected from $C_{1-8}$alkyl, $C_{1-8}$alkylaryl or $C_{6-14}$aryl; or $R^2$ and $R^3$ can together form a 3- to 8-membered ring;

$R^4$ is -OR$^8$, -NR$^8$R$^9$ or -SR$^8$, where $R^8$ and $R^9$, which may be the same or different, are independently selected from hydrogen, or $C_{1-8}$alkyl, $C_{3-8}$cycloalkyl, $C_{2-8}$alkenyl, $C_{5-8}$cycloalkenyl, or aralkyl, wherein each $C_{1-8}$alkyl, $C_{3-8}$cycloalkyl, $C_{2-8}$alkenyl, $C_{5-8}$cycloalkenyl, or aralkyl may be optionally substituted with one or more substituents selected from the group consisting of halo, hydroxy, alkoxy, amino, aminoalkyl, aminodialkyl, -SH, thioalkyl, carboxylate and esters thereof, carboxyalkyl, -CONHR$^6$, and -CONR$^6$R$^7$, wherein $R^6$ and $R^7$, which may be the same or different, are independently selected from $C_{1-8}$alkyl, $C_{1-8}$alkylaryl or $C_{6-14}$aryl;

$R^5$ is hydrogen; $C_{1-8}$alkyl; or $C_{6-14}$aryl; or $R^2$ and $R^5$ may together form a 5- or 6-membered ring; or $R^3$ and $R^5$ may together form a 5- or 6-membered ring;

X is $C_{1-6}$alkoxy, optionally substituted by $C_{3-6}$cycloalkyl; $C_{3-8}$cycloalkyloxy; aryloxy, aralkyl or aralkyloxy in which the aryl may optionally be substituted with $C_{1-8}$alkyl, hydroxy, or halogen; $C_{3-6}$cycloalkylthio; $C_{1-8}$alkylthio; arylthio, or aralkylthio in which the aryl may optionally be substituted with $C_{1-8}$alkyl, hydroxy, or halogen; or X is a heterocyclic group containing an oxygen atom or one or two nitrogen atoms, and 3-7 carbon atoms with optional double bonds in the ring, optionally containing a sulfur and/or oxygen heteroatom and optionally substituted on the ring by one or more $C_{1-8}$alkyl, hydroxy or halogen groups, $C_{3-6-}$cycloalkylthio, aralkylthio in which the aryl may be substituted with $C_{1-8}$alkyl, hydroxy or halogen; or X represents an imidazolylthio group in which the imidazolyl moiety may be substituted with $C_{1-8}$alkyl and/or C substituted with nitro; or X represents an amino group which is mono- or di-substituted by $C_{1-8}$alkyl, $C_{1-6}$alkoxy, hydroxy$_{C1-8}$alkyl and/or $C_{3-6}$cycloalkyl, $C_{6-14}$aryl, aralkyl, in which the $C_{6-14}$aryl may be optionally substituted with $C_{1-8}$alkyl, hydroxy, halogen or allyl optionally substituted with mono- or di-alkyl or alkoxy groups;

or a pharmaceutically acceptable derivative thereof; provided that X cannot be amino monosubstituted with cyclopropyl.

**2.** A compound of formula (I) according to claim 1 wherein $R^1$ is $C_{6-14}$aryl; $R^2$ and $R^3$ are independently selected from hydrogen, $C_{1-8}$alkyl, $C_{6-14}$aryl or aralkyl optionally substituted with $C_{1-8}$alkyl; $R^4$ is -OR$^8$, where $R^8$ is selected

from $C_{1-8}$alkyl or aralkyl; $R^5$ is hydrogen; and X represents $C_{1-6}$alkoxy or $C_{1-6}$alkylthio; or X represents a heterocyclic group containing a nitrogen atom, and 3-7 carbon atoms; or X represents an amino group which is mono- or di-substituted by $C_{1-6}$alkyl, $C_{1-6}$alkoxy, hydroxy$C_{1-6}$alkyl and/or $C_{3-6}$cycloalkyl, $C_{6-14}$aryl, aralkyl, in which the aryl may be optionally substituted with $C_{1-8}$alkyl, hydroxy, halogen or allyl optionally substituted with mono- or dialkyl or alkoxy groups; or a pharmaceutically acceptable derivative thereof provided that X cannot be amino monosubstituted with cyclopropyl.

**3.** A compound as claimed in claim 1 selected from the group consisting of
(1S,*cis*)-4-[2-Amino-6-(1-azetidinyl)-9H-purin-9-yl]-2-cyclopentene-1-methanol O-[phenyl(methoxy-L-alaninyl)] phosphoramidate;
(1S,*cis*)-4-[2-Amino-6-(cyclopropylmethylamino)-9H-purin-9-yl]-2-cyclopentene-1-methanol O-[phenyl(methoxy-L-alaninyl)]phosphoramidate;
(1S,*cis*)-4-(2-Amino-6-butoxy-9H-purin-9-yl)-2-cyclopentene-1-methanol O-[phenyl(methoxy-L-alaninyl)]phosphoramidate;
(1S,*cis*)-4-(2-Amino-6-methoxy-9H-purin-9-yl)-2-cyclopentene-1-methanol O-[phenyl(methoxy-L-alaninyl)]phosphoramidate;
(1S,*cis*)-4-[2-Amino-6-(propylamino)-9H-purin-9-yl]-2-cyclopentene-1-methanol O-[phenyl(methoxy-L-alaninyl)] phosphoramidate;
(1S,*cis*)-4-[2-Amino-6-(isopropylamino)-9H-purin-9-yl]-2-cyclopentene-1-methanol O-[phenyl(methoxy-L-alaninyl)]phosphoramidate;
(1S,*cis*)-4-[2-Amino-6-(allylthio)-9H-purin-9-yl]-2-cyclopentene-1-methanol O-[phenyl(methoxy-L-alaninyl)]phosphoramidate;
(1S,*cis*)-4-(2,6-Diamino-9H-purin-9-yl)-2-cyclopentene-1-methanol O-[phenyl(methoxy-L-alaninyl)]phosphoramidate;
(1S,*cis*)-4-[2-Amino-6-(cyclopropylmethylamino)-9H-purin-9-yl]-2-cyclopentene-1-methanol O-[phenyl(methoxy-$\alpha,\alpha$-dimethylglycinyl)]phosphoramidate;
(1S,*cis*)-4-[2-Amino-6-(cyclopropylmethylamino)-9H-purin-9-yl]-2-cyclopentene-1-methanol O-[phenyl(benzyloxy-L-alaninyl)]phosphoramidate;
(1S,*cis*)-4-[2-Amino-6-(cyclopropylmethylamino)-9H-purin-9-yl]-2-cyclopentene-1-methanol O-[phenyl(methoxy-L-phenylalaninyl)]phosphoramidate;
and pharmaceutically acceptable derivatives thereof.

**4.** A compound according to any of claims 1 to 3 in the form of a single isomer.

**5.** A compound according to any of claims 1 to 3 in the form of a mixture of diastereomers.

**6.** A compound according to any of claims 1 to 3 wherein the pharmaceutically acceptable derivative is a salt.

**7.** A pharmaceutical composition comprising an effective anti-viral amount of a compound of formula (I) according to any of claims 1 to 3 or a pharmaceutically acceptable derivative thereof together with a pharmaceutically acceptable carrier therefor.

**8.** The pharmaceutical composition according to claim 7, further comprising an antiviral agent other than a compound of formula (I).

**9.** A pharmaceutical composition according to claim 7 or 8 in the form of a tablet or capsule.

**10.** A pharmaceutical composition according to claim 7 or 8 in the form of a solution, suspension, or syrup.

**11.** A compound of formula (I) as claimed in claim 1 for use in medical therapy.

**12.** Use of a compound of formula (1) as claimed in claim 1 in the manufacture of a medicament for the treatment or prophylaxis of a virus infection.

**13.** Use as claimed in claim 12 wherein the virus is Human Immunodeficiency Virus or hepatitis B virus.

## Patentansprüche

**1.** Verbindung der Formel (I)

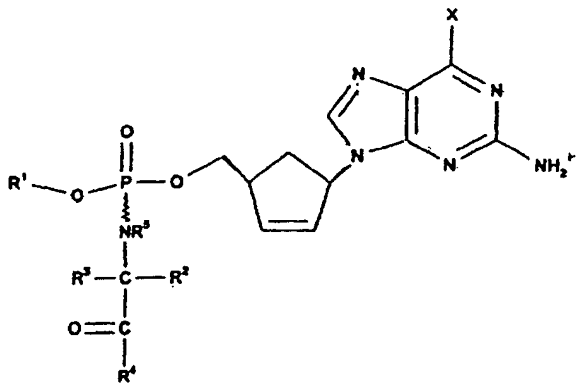

(I)

wobei:

$R^1$ ein Wasserstoffatom; $C_{6-14}$-Arylrest; oder Heteroarylrest ist, welcher gegebenenfalls mit einem oder mehreren Substituenten, ausgewählt aus einem $C_{1-6}$-Alkoxyrest, einer Nitrogruppe, einem Halogenatom, einer Amino-, Hydroxygruppe, einem Carboxylat und Estern davon, einem Carboxyalkylrest, -CONHR$^6$ und -CONR$^6$R$^7$, substituiert ist, wobei $R^6$ und $R^7$, welche gleich oder verschieden sein können, unabhängig voneinander aus einem $C_{1-8}$-Alkyl-, $C_{1-8}$-Alkylaryl- oder $C_{6-14}$-Arylrest ausgewählt sind;

$R^2$ und $R^3$ unabhängig voneinander aus einem Wasserstoffatom oder $C_{1-8}$-Alkyl-, $C_{3-8}$-Cycloalkyl-, $C_{2-8}$-Alkenyl-, $C_{5-8}$-Cycloalkenyl-, $C_{6-14}$-Aryl- oder Aralkylrest ausgewählt sind, wobei jeder $C_{1-8}$-Alkyl-, $C_{3-8}$Cycloalkyl-, $C_{2-8}$-Alkenyl-, $C_{5-8}$-Cycloalkenyl-, $C_{6-14}$-Aryl- oder Aralkylrest gegebenenfalls mit einem oder mehreren Substituenten, ausgewählt aus einem $C_{1-8}$-Alkylrest, einem Halogenatom, einer Hydroxygruppe, einem Alkoxyrest, einer Aminogruppe, einem Aminoalkyl-, Aminodialkylrest, einem Rest -SH, einem Thioalkylrest, Carboxylat und Estern davon, einem Carboxyalkylrest, -CONHR$^6$ und -CONR$^6$R$^7$, substituiert sein kann, wobei $R^6$ und $R^7$, welche gleich oder verschieden sein können, unabhängig voneinander aus einem $C_{1-8}$-Alkyl-, $C_{1-8}$-Alkylaryl- oder $C_{6-14}$-Arylrest ausgewählt sind; oder $R^2$ und $R^3$ zusammen einen 3- bis 8-gliedrigen Ring bilden können;

$R^4$ ein Rest -OR$^8$, -NR$^8$R$^9$ oder -SR$^8$ ist, wobei $R^8$ und $R^9$, welche gleich oder verschieden sein können, unabhängig voneinander aus einem Wasserstoffatom oder $C_{1-8}$-Alkyl-, $C_{3-8}$-Cycloalkyl-, $C_{2-8}$-Alkenyl-, $C_{5-8}$-Cycloalkenyl- oder Aralkylrest ausgewählt sind, wobei jeder $C_{1-8}$-Alkyl-, $C_{3-8}$-Cycloalkyl-, $C_{2-8}$-Alkenyl-, $C_{5-8}$-Cycloalkenyl- oder Aralkylrest gegebenenfalls mit einem oder mehreren Substituenten, ausgewählt aus einem Halogenatom, einer Hydroxygruppe, einem Alkoxyrest, einer Aminogruppe, einem Aminoalkyl-, Aminodialkylrest, einem Rest -SH, einem Thioalkylrest, Carboxylat und Estern davon, einem Carboxyalkylrest, -CONHR$^6$ und -CONR$^6$R$^7$, substituiert sein kann, wobei $R^6$ und $R^7$, welche gleich oder verschieden sein können, unabhängig voneinander aus einem $C_{1-8}$-Alkyl-, $C_{1-8}$-Alkylaryl- oder $C_{6-14}$-Arylrest ausgewählt sind;

$R^5$ ein Wasserstoffatom; ein $C_{1-8}$-Alkyl- oder $C_{6-14}$-Arylrest ist; oder $R^2$ und $R^5$ zusammen einen 5- oder 6-gliedrigen Ring bilden können oder $R^3$ und $R^5$ zusammen einen 5- oder 6-gliedrigen Ring bilden können;

X ein $C_{1-6}$-Alkoxyrest ist, welcher gegebenenfalls mit einem $C_{3-6}$-Cycloalkyl-, $C_{3-8}$-Cycloalkyloxy-, Aryloxy-, Aralkyl- oder Aralkyloxyrest, wobei der Arylrest gegebenenfalls mit einem $C_{1-8}$-Alkylrest, einer Hydroxygruppe oder einem Halogenatom substituiert sein kann; $C_{3-6}$-Cycloalkylthio-; $C_{1-8}$-Alkylthio-; Arylthio- oder Aralkylthiorest, wobei der Arylrest gegebenenfalls mit einem $C_{1-8}$-Alkylrest, einer Hydroxygruppe oder einem Halogenatom substituiert sein kann; substituiert ist; oder X ein heterocyclischer Rest ist, welcher ein Sauerstoffatom oder ein oder zwei Stickstoffatome und 3 bis 7 Kohlenstoffatome, gegebenenfalls mit Doppelbindungen im Ring, enthält, gegebenenfalls ein Schwefel- und/oder ein Sauerstoffheteroatom enthält und gegebenenfalls am Ring mit einem oder mehreren $C_{1-8}$-Alkylresten, Hydroxygruppen oder Halogenatomen, $C_{3-6}$-Cycloalkylthio-, Aralkylthioresten, wobei der Arylrest mit einem $C_{1-8}$-Alkylrest, einer Hydroxygruppe oder einem Halogenatom substituiert sein kann, substituiert ist; oder X eine Imidazolylthiogruppe bedeutet, wobei die Imidazolyleinheit mit einem $C_{1-8}$-Alkylrest substituiert sein kann und/oder mit einer Nitrogruppe C-substituiert sein kann; oder X eine Aminogruppe bedeutet, welche mit einem $C_{1-8}$-Alkyl-, $C_{1-6}$-Alkoxy-, Hydroxy-$C_{1-8}$-alkyl- und/

oder $C_{3-6}$-Cycloalkyl-, $C_{6-14}$-Aryl-, Aralkylrest, wobei der $C_{6-14}$-Arylrest gegebenenfalls mit einem $C_{1-8}$-$C_{1-8}$-Alkylrest, einer Hydroxygruppe, einem Halogenatom oder einem Allylrest, gegebenenfalls substituiert mit einem Mono- oder Dialkyl- oder Alkoxyrest, substituiert sein kann, mono- oder disubstituiert ist;

oder ein pharmazeutisch verträgliches Derivat davon; mit der Maßgabe, dass X keine mit einer Cyclopropylgruppe monosubstituierte Aminogruppe sein kann.

**2.** Verbindung der Formel (I) gemäß Anspruch 1, wobei $R^1$ ein $C_{6-14}$-Arylrest ist; $R^2$ und $R^3$ unabhängig voneinander aus einem Wasserstoffatom, $C_{1-8}$-Alkyl-, $C_{6-14}$-Aryl- oder Aralkylrest, gegebenenfalls substituiert mit einem $C_{1-8}$-Alkylrest, ausgewählt sind; $R^4$ ein Rest -$OR^8$ ist, wobei $R^8$ aus einem $C_{1-8}$-Alkyl- oder Aralkylrest ausgewählt ist; $R^5$ ein Wasserstoffatom ist und X einen $C_{1-6}$-Alkoxy- oder $C_{1-6}$-Alkylthiorest bedeutet; oder X einen heterocyclischen Rest bedeutet, welcher ein Stickstoffatom und 3 bis 7 Kohlenstoffatome enthält; oder X eine Aminogruppe bedeutet, welche mit einem $C_{1-6}$-Alkyl-, $C_{1-6}$-Alkoxy-, Hydroxy-$C_{1-6}$-alkyl- und/oder $C_{3-6}$-Cycloalkyl-, $C_{6-14}$-Aryl-, Aralkylrest, wobei der Arylrest gegebenenfalls mit einem $C_{1-8}$-Alkylrest, einer Hydroxygruppe, einem Halogenatom oder einem Allylrest, gegebenenfalls substituiert mit einem Mono- oder Dialkyl- oder Alkoxyrest, substituiert sein kann, mono- oder disubstituiert ist; oder ein pharmazeutisch verträgliches Derivat davon; mit der Maßgabe, dass X keine mit einer Cyclopropylgruppe monosubstituierte Aminogruppe sein kann.

**3.** Verbindung gemäß Anspruch 1, ausgewählt aus
(1S,cis)-4-[2-Amino-6-(1-azetidinyl)-9H-purin-9-yl]-2-Cyclopenten-1-methanol O-[phenyl(methoxy-L-alaninyl)] phosphoramidat;
(1S,cis)-4-[2-Amino-6-(cyclopropylmethylamino)-9H-purin-9-yl]-2-cyclopenten-1-methanol O-[phenyl(methoxy-L-alaninyl)]phosphoramidat;
(1S,cis)-4-(2-Amino-6-butoxy-9H-purin-9-yl)-2-cyclopenten-1-methanol O-[phenyl(methoxy-L-alaninyl)]phosphoramidat;
(1S,cis)-4-(2-Amino-6-methoxy-9H-purin-9-yl)-2-cyclopenten-1-methanol O-[phenyl(methoxy-L-alaninyl)]phosphoramidat;
(1S,cis)-4-[2-Amino-6-(propylamino)-9H-purin-9-yl]-2-cyclopenten-1-methanol O-[phenyl(methoxy-L-alaninyl)] phosphoramidat;
(1S,cis)-4-[2-Amino-6-(isopropylamino)-9H-purin-9-yl]-2-cyclopenten-1-methanol O-[phenyl(methoxy-L-alaninyl)] phosphoramidat;
(1S,cis)-4-[2-Amino-6-(allylthio)-9H-purin-9-yl]-2-cyclopenten-1-methanol O-[phenyl(methoxy-L-alaninyl)]phosphoramidat;
(1S,cis)-4-(2,6-Diamino-9H-purin-9-yl)-2-cyclopenten-1-methanol O-[phenyl(methoxy-L-alaninyl)]phosphoramidat;
(1S,cis)-4-[2-Amino-6-(cyclopropylmethylamino)-9H-purin-9-yl]-2-cyclopenten-1-methanol O-[phenyl(methoxy-α,α-dimethylglycinyl)]phosphoramidat;
(1S,cis)-4-[2-Amino-6-(cyclopropylmethylamino)-9H-purin-9-yl)-2-cyclopenten-1-methanol O-[phenyl(benzyloxy-L-alaninyl)]phosphoramidat;
(1S,cis)-4-[2-Amino-6-(cyclopropylmethylamino)-9H-purin-9-yl]-2-cyclopenten-1-methanol O-[phenyl(methoxy-L-phenylalaninyl)]phosphoramidat;
und pharmazeutisch verträgliche Derivate davon.

**4.** Verbindung gemäß einem der Ansprüche 1 bis 3 in Form eines einzigen Isomers.

**5.** Verbindung gemäß einem der Ansprüche 1 bis 3 in Form eines Diastereomerengemisches.

**6.** Verbindung gemäß einem der Ansprüche 1 bis 3, wobei das pharmazeutisch verträgliche Derivat ein Salz ist.

**7.** Arzneimittel, umfassend eine antiviral wirksame Menge einer Verbindung der Formel (I) gemäß einem der Ansprüche 1 bis 3 oder ein pharmazeutisch verträgliches Derivat davon zusammen mit einem pharmazeutisch verträglichen Träger dafür.

**8.** Arzneimittel gemäß Anspruch 7, weiterhin umfassend ein Antivirenmittel verschieden von einer Verbindung der Formel (I).

**9.** Arzneimittel gemäß Anspruch 7 oder 8 in Form einer Tablette oder Kapsel.

**10.** Arzneimittel gemäß Anspruch 7 oder 8 in Form einer Lösung, Suspension oder eines Sirups.

**11.** Verbindung der Formel (1) gemäß Anspruch 1 zur Verwendung in der medizinischen Therapie.

**12.** Verwendung einer Verbindung der Formel (I) gemäß Anspruch 1 zur Herstellung eines Medikaments zur Behandlung oder Vorbeugung einer Virusinfektion.

**13.** Verwendung gemäß Anspruch 12, wobei der Virus ein HIV- (Human Immunodeficiency Virus) oder Hepatitis B-Virus ist.

**Revendications**

**1.** Composé de formule (I)

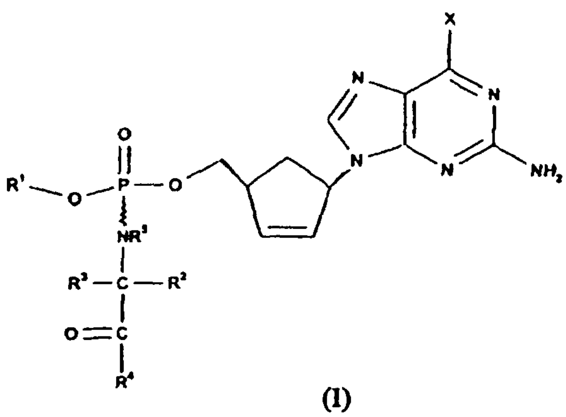

(I)

dans laquelle :

$R^1$ représente un atome d'hydrogène ; un groupe aryle en $C_6$ à $C_{14}$ ; ou hétéroaryle, facultativement substitué avec un ou plusieurs substituants choisis dans le groupe consistant en des substituants alkoxy en $C_1$ à $C_6$, nitro, halogéno, amino, hydroxy, carboxylate et ses esters, carboxyalkyle, -$CONHR^6$, et -$CONHR^6R^7$, dans lesquels $R^6$ et $R^7$, qui peuvent être identiques ou différents, sont choisis indépendamment entre des groupes alkyle en $C_1$ à $C_8$, (alkyle en $C_1$ à $C_8$) aryle et aryle en $C_6$ à $C_{14}$ ;
$R^2$ et $R^3$ sont choisis indépendamment entre un atome d'hydrogène, des groupes alkyle en $C_1$ à $C_8$, cycloalkyle en $C_3$ à $C_8$, alcényle en $C_2$ à $C_8$, cycloalcényle en $C_5$ à $C_8$, aryle en $C_6$ à $C_{14}$ ou aralkyle, dans lesquels chaque groupe alkyle en $C_1$ à $C_8$, cycloalkyle en $C_3$ à $C_8$, alcényle en $C_2$ à $C_8$, cycloalcényle en $C_5$ à $C_8$, aryle en $C_6$ à $C_{14}$ ou aralkyle peut être facultativement substitué avec un ou plusieurs substituants choisis dans le groupe consistant en des substituants alkyle en $C_1$ à $C_8$, halogéno, hydroxy, alkoxy, amino, aminoalkyle, aminodialkyle, -SH, thioalkyle, carboxylate et ses esters, carboxyalkyle, -$CONHR^6$ et -$CONR^6R^7$, dans lesquels $R^6$ et $R^7$, qui peuvent être identiques ou différents, sont choisis indépendamment entre des groupes alkyle en $C_1$ à $C_8$, (alkyle en $C_1$ à $C_8$) aryle et aryle en $C_6$ à $C_{14}$ ; ou bien $R^2$ et $R^3$ peuvent, conjointement, former un noyau tri- à octogonal ;
$R^4$ représente un groupe -$OR^8$, -$NR^8R^9$ ou -$SR^8$, dans lequel $R^8$ et $R^9$, qui peuvent être identiques ou différents, sont choisis indépendamment entre un atome d'hydrogène, des groupes alkyle en $C_1$ à $C_8$, cycloalkyle en $C_3$ à $C_8$, alcényle en $C_2$ à $C_8$, cycloalcényle en $C_5$ à $C_8$ ou aralkyle, dans lesquels chaque groupe alkyle en $C_1$ à $C_8$, cycloalkyle en $C_3$ à $C_8$, alcényle en $C_2$ à $C_8$, cycloalcényle en $C_5$ à $C_8$ ou aralkyle peut être facultativement substitué avec un ou plusieurs substituants choisis dans le groupe consistant en des substituants halogéno, hydroxy, alkoxy, amino, aminoalkyle, aminodialkyle, -SH, thioalkyle, carboxylate et ses esters, carboxyalkyle, -$CONHR^6$ et -$CONR^6R^7$, dans lesquels $R^6$ et $R^7$, qui peuvent être identiques ou différents, sont choisis indépendamment entre des groupes alkyle en $C_1$ à $C_8$, (alkyle en $C_1$ à $C_8$) aryle et aryle en $C_6$ à $C_{14}$ ;
$R^5$ représente un atome d'hydrogène, un groupe alkyle en $C_1$ à $C_8$ ou aryle en $C_6$ à $C_{14}$ ; ou bien $R^2$ et $R^5$ peuvent, conjointement, former un noyau penta- ou hexagonal ; ou bien $R^3$ et $R^5$ peuvent, conjointement, former un noyau penta- ou hexagonal ;
X représente un groupe alkoxy en $C_1$ à $C_6$, facultativement substitué avec un substituant cycloalkyle en $C_3$ à

$C_6$ ;
cycloalkyloxy en $C_3$ à $C_8$ ; aryloxy, aralkyle ou aralkyloxy dans lequel le groupe aryle peut être facultativement substitué avec un substituant alkyle en $C_1$ à $C_8$, hydroxy ou halogène ; cycloalkylthio en $C_3$ à $C_6$ ; alkylthio en $C_1$ à $C_8$, arylthio, ou aralkylthio dans lequel le groupe aryle peut être facultativement substitué avec un substituant alkyle en $C_1$ à $C_8$, hydroxy ou halogène ; ou bien X représente un groupe hétérocyclique contenant un atome d'oxygène ou 1 ou 2 atomes d'azote et 3 à 7 atomes de carbone avec des doubles liaisons facultatives dans le noyau, contenant facultativement un hétéroatome de soufre et/ou d'oxygène et facultativement substitué sur le noyau avec un ou plusieurs groupes alkyle en $C_1$ à $C_8$, hydroxy ou halogéno, cycloalkylthio en $C_3$ à $C_6$, aralkylthio dans lequel le groupe aryle peut être substitué avec un substituant alkyle en $C_1$ à $C_8$, hydroxy ou halogéno ; ou bien X représente un groupe imidazolylthio dans lequel le groupement imidazolyle peut être substitué avec un substituant alkyle en $C_1$ à $C_8$ et/ou C-substitué avec un substituant nitro ; ou bien X représente un groupe amino qui est mono- ou di-substitué avec un substituant alkyle en $C_1$ à $C_8$, alkoxy en $C_1$ à $C_6$, hydroxyalkyle en $C_1$ à $C_8$ et/ou cycloalkyle en $C_3$ à $C_6$, aryle en $C_6$ à $C_{14}$, aralkyle, dans lesquels le groupe aryle en $C_6$ à $C_{14}$ peut être facultativement substitué avec un substituant alkyle en $C_1$ à $C_8$, hydroxy, halogéno ou allyle facultativement substitué avec des groupes mono- ou di-alkyle ou alkoxy ;

ou un de ses dérivés pharmaceutiquement acceptables ; sous réserve que X ne puisse représenter un groupe amino monosubstitué avec un substituant cyclopropyle.

**2.** Composé de formule (I) suivant la revendication 1, dans lequel $R^1$ représente un groupe aryle en $C_6$ à $C_{14}$ ; $R^2$ et $R^3$ sont choisis indépendamment entre un atome d'hydrogène, des groupes alkyle en $C_1$ à $C_8$, aryle en $C_6$ à $C_{14}$ ou aralkyle facultativement substitué avec un substituant alkyle en $C_1$ à $C_8$ ; $R^4$ représente un groupe $-OR^8$, dans lequel $R^8$ est choisi entre un groupe alkyle en $C_1$ à $C_8$ et un groupe aralkyle ; $R^5$ représente un atome d'hydrogène ; et X représente un groupe alkoxy en $C_1$ à $C_6$ ou alkylthio en $C_1$ à $C_6$ ; ou bien X représente un groupe hétérocyclique contenant un atome d'azote et 3 à 7 atomes de carbone ; ou bien X représente un groupe amino qui est mono- ou di- substitué avec un substituant alkyle en $C_1$ à $C_6$, alkoxy en $C_1$ à $C_6$, hydroxyalkyle en $C_1$ à $C_6$ et/ou cycloalkyle en $C_3$ à $C_6$, aryle en $C_6$ à $C_{14}$, aralkyle, dans lesquels le groupe aryle peut être facultativement substitué avec un substituant alkyle en $C_1$ à $C_8$, hydroxy, halogène ou allyle facultativement substitué avec des groupes mono- ou di- alkyle ou alkoxy ; ou un de ses dérivés pharmaceutiquement acceptables, sous réserve que X ne puisse représenter un groupe amino monosubstitué avec un substituant cyclopropyle.

**3.** Composé suivant la revendication 1, choisi dans le groupe consistant en les composés suivants :

O-[phényl(méthoxy-L-alaninyl)]phosphoramidate de (1S,*cis*)-4-[2-amino-6-(1-azétidinyl)-9H-purine-9-yle]-2-cyclopentène-1-méthanol ;
O-[phényl(methoxy-L-alaninyl)]phosphoramidate de (1S, *cis*)-4-[2-amino-6-(cyclopropylméthylamino)-9H-purine-9-yl]-2-cyclopentène-1-méthanol ;
O-[phényl(méthoxy-L-alaninyl)]phosphoramidate de (1S,*cis*)-4-[2-amino-6-butoxy-9H-purine-9-yl]-2-cyclopentène-1-méthanol ;
O-[phényl(méthoxy-L-alaninyl)]phosphoramidate de (1S,*cis*)-4-[2-amino-6-méthoxy-9H-purine-9-yl]-2-cyclopentène-1-méthanol ;
O-[phényl(méthoxy-L-alaninyl)]phosphoramidate de *(*1S*,cis)*-4-[2-amino-6-(propylamino)-9H-purine-9-yl]-2-cyclopentène-1-méthanol ;
O-[phényl(méthoxy-L-alaninyl)]phosphoramidate de (1S,*cis*)-4-[2-amino-6-(isopropylamino)-9H-purine-9-yl]-2-cyclopentène-1-méthanol ;
O-[phényl(méthoxy-L-alaninyl)]phosphoramidate de (1S,*cis*)-4-[2-amino-6-(allylthio)-9H-purine-9-yl]-2-cyclopentène-1-méthanol ;
O-[phényl (méthoxy-L-alaninyl)]phosphoramidate de (1S,*cis*)-4-[2,6-diamino-9H-purine-9-yl]-2-cyclopentène-1-méthanol ;
O-[phényl (méthoxy-α,α-diméthylglycinyl)]phosphoramidate de (1S,*cis*)-4-[2-amino-6-(cyclopropylméthylamino)-9H-purine-9-yl]-2-cyclopentène-1-méthanol ;
O-[phényl(benzyloxy-L-alaninyl)]phosphoramidate de (1S,*cis*)-4-[2-amino-6-(cyclopropylméthylamino)-9H-purine-9-yl]-2-cyclopentène-1-méthanol ;
O-[phényl(méthoxy-L-phénylalaninyl)]phosphoramidate de (1S,*cis*)-4-[2-amino-6-(cyclopropylméthylamino)-9H-purine-9-yl]-2-cyclopentène-1-méthanol ;

et leurs dérivés pharmaceutiquement acceptables.

4. Composé suivant l'une quelconque des revendications 1 à 3, sous forme d'un isomère unique.

5. Composé suivant l'une quelconque des revendications 1 à 3, sous forme d'un mélange de diastéréoisomères.

6. Composé suivant l'une quelconque des revendications 1 à 3, dans lequel le dérivé pharmaceutiquement acceptable est un sel.

7. Composition pharmaceutique comprenant une quantité antivirale efficace d'un composé de formule (I) suivant l'une quelconque des revendications 1 à 3 ou d'un de ses dérivés pharmaceutiquement acceptables, conjointement avec un support pharmaceutiquement acceptable à cette fin.

8. Composition pharmaceutique suivant la revendication 7, comprenant en outre un agent antiviral autre qu'un composé de formule (I).

9. Composition pharmaceutique suivant la revendication 7 ou 8, sous forme d'un comprimé ou d'une capsule.

10. Composition pharmaceutique suivant la revendication 7 ou 8, sous forme d'une solution, d'une suspension ou d'un sirop.

11. Composé de formule (I) suivant la revendication 1, destiné à être utilisé en thérapie médicale.

12. Utilisation d'un composé de formule (I) suivant la revendication 1 dans la production d'un médicament destiné au traitement ou à la prophylaxie d'une infection virale.

13. Utilisation suivant la revendication 12, dans laquelle le virus est le virus de l'immunodéficience humaine ou le virus de l'hépatite B.